(19) **Europäisches Patentamt / European Patent Office / Office européen des brevets**

(11) **EP 4 058 074 B1**

(12) **EUROPEAN PATENT SPECIFICATION**

(45) Date of publication and mention
of the grant of the patent:
**25.02.2026 Bulletin 2026/09**

(21) Application number: **20821532.7**

(22) Date of filing: **16.11.2020**

(51) International Patent Classification (IPC):
*A61L 27/18* (2006.01)     *A61L 27/20* (2006.01)
*A61L 27/24* (2006.01)     *A61L 27/38* (2006.01)
*A61L 27/50* (2006.01)     *A61L 27/54* (2006.01)
*B33Y 10/00* (2015.01)     *B33Y 80/00* (2015.01)
*D01D 5/00* (2006.01)      *A61L 27/34* (2006.01)
*A61L 27/56* (2006.01)     *C12M 3/00* (2006.01)
*C12M 1/00* (2006.01)

(52) Cooperative Patent Classification (CPC):
(C-Sets available)
**A61L 27/18; A61L 27/20; A61L 27/34;
A61L 27/3834; A61L 27/507; A61L 27/54;
A61L 27/56; B33Y 10/00; B33Y 80/00;
C12M 21/08; C12M 23/38; C12M 25/14;
C12M 29/10;** A61L 2300/222; A61L 2300/41;
(Cont.)

(86) International application number:
**PCT/US2020/060717**

(87) International publication number:
**WO 2021/097419 (20.05.2021 Gazette 2021/20)**

(54) **SYSTEMS AND METHODS PRODUCING SEEDED GRAFTS**

SYSTEME UND VERFAHREN ZUM PRODUZIEREN VON GEIMPFTEN TRANSPLANTATEN

SYSTÈMES ET PROCÉDÉS DE PRODUCTION DE GREFFONS ENSEMENCÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.11.2019 US 201962936225 P**

(43) Date of publication of application:
**21.09.2022 Bulletin 2022/38**

(73) Proprietor: **Research Institute at Nationwide
Children's Hospital
Columbus, Ohio 43205 (US)**

(72) Inventors:
• **BREUER, Christopher
Columbus, Ohio 43205 (US)**

• **BEST, Cameron
Columbus, Ohio 43205 (US)**
• **STROUSE, Robert
Columbus, Ohio 43205 (US)**
• **REINHARDT, James
Columbus, Ohio 43205 (US)**

(74) Representative: **Potter Clarkson
Chapel Quarter
Mount Street
Nottingham NG1 6HQ (GB)**

(56) References cited:
**WO-A2-2012/006072     US-A1- 2018 353 649**

**(Cont. next page)**

(52) Cooperative Patent Classification (CPC): (Cont.)
A61L 2300/414; A61L 2300/416; A61L 2300/426;
D01D 5/0076

C-Sets
**A61L 27/18, C08L 67/04;**
**A61L 27/18, C08L 77/00;**
**A61L 27/18, C08L 85/02**

## Description

### FIELD OF THE INVENTION

The present invention is generally in the field of tissue engineered grafts, particularly those that are seeded with cells and are designed to be absorbed and replaced by patient's own tissues, and methods of making.

### BACKGROUND OF THE INVENTION

Surgical treatment of many complex congenital cardiac anomalies involves implantation of synthetic scaffolds of materials such as GORE-TEX® and DACRON®. A common example of such an application is the total cavopulmonary connection (TCPC) for single ventricle anomalies. In some instances, the use of these synthetic grafts as scaffolds is complicated by progressive obstruction, susceptibility to infection, and risk of thromboembolic complications. In all instances, a significant limitation to cardiovascular reconstruction is lack of growth potential of synthetic implants. For TCPC, this may lead to sub-optimal management strategies including either postponement of completion of the Fontan circulation because of patient size, or over-sizing of scaffolds, which results in sub-optimal flow characteristics such as expiratory phase back-flow and regions of flow stagnation.

Tissue-engineered vascular grafts (TEVGs) offer the potential to overcome these problems by providing a biodegradable scaffold in which autologous cells proliferate and mature into a physiologically functional blood vessel as scaffold polymers degrade (Shin'oka, et al. N. Engl. J. Med. 344(7), 532-533 (2001); Hibino, et al. J Thorac Cardiovasc Surg. 139(2), 431-436.e432 (2010 Roh JD, et al. Biomaterials. 29(10), 1454-1463 (2008); Hibino, et al. FASEB J. 25(8), 2731-2739 (2011); Hibino, et al. FASEB J. 25(12), 4253-4263 (2011); Kurobe, et al. Tissue Eng Part C Methods 21(1), 88-93 (2015)).

Clinical trials in humans confirmed the growth capacity of the TEVG and demonstrated no graft related deaths or graft failures (Shin'oka, et al. J Thorac Cardiovasc Surg. 129(6), 1330-1338 (2005)). However, the results of this study also demonstrated that stenosis was the primary graft-related complication, effecting nearly 25% of graft recipients, with 16% of recipients developing critical stenosis (>75% decrease in luminal diameter) (Hibino, et al. J Thorac Cardiovasc Surg. 139(2), 431-436.e432 (2010)). Despite promising results using TEVGs for the treatment of patients with congenital heart diseases, the high incidence of graft stenosis in clinical applications hinders wide spread use of this technology (Fernandez, et al. Current opinion in chemical engineering 3, 83-90 (2014); Mcallister, et al. Lancet 373(9673), 1440-1446 (2009); Wystrychowski, et al. J Vasc Surg 60(5), 1353-1357 (2014)).

Moreover, before routine clinical use of the TEVGs can be recommended, the assembly of the TEVG must be optimized to personalize the grafts and minimize the time required to make the graft and improve its overall utility (Patterson, et al. Regenerative Medicine 7(3), 409-419 (2012)).

US Patent No. 9,090,863 and U.S. Publication No. US 2018/0353649 describe closed disposable seeding systems (CDSS) for seeding scaffolds and grafts with cells. The CDSS includes seeding chambers for housing and seeding the scaffolds and grafts with cells. The seeding of scaffolds and grafts using the CDSS of US Patent No. 9,090,863 and U.S. Publication No. US 2018/0353649 provide a non-uniform cell seeding density along the length of the scaffolds and grafts.

There remains a need for improved seeded scaffolds and grafts having a uniform cell density along the length of the scaffolds and grafts. There remains a need for scaffolds and grafts inducing reversible stenosis following implantation.

Therefore, it is the object of the present invention to provide improved seeding chambers for use with the CDSS for uniform cell seeding along the length of the scaffolds and grafts to reduce the rates of or prevent stenosis following implantation.

It is another object of the present invention to provide a system with improved seeding chambers for seeding cells on scaffolds and grafts to reduce the rates of, prevent, or reverse stenosis following implantation.

It is yet another object of the present invention to provide a method for seeding cells on scaffolds and grafts to reduce the rates of, prevent, or reverse stenosis following implantation.

### SUMMARY OF THE INVENTION

It was discovered during clinical trials that scaffolds and grafts having non-uniform cell seeding density along the length of the scaffold or graft lead to an increased incidence of graft stenosis following implantation. Improved seeding chambers for use in a closed disposable seeding system have been developed which provide a uniform density of cells seeded along the length of the scaffold or graft. The cell seeding chamber, termed "flip" chamber and "capacitor" chamber, have a cap with lateral ports.

The present invention focusses on the "flip" chamber development.

In a first aspect, there is provided a cell seeding chamber for use in a closed disposable cell seeding system, the cell seeding chamber comprising: a housing having a variable width and a length, a cap comprising an opening for a suction rod insertable into the housing, and one or more lateral fluid ports, and for securing a perforated or porous mandrel that can be positioned over the suction rod, and a base, wherein the housing has a bulge positioned between about 30% and 60% of the length of the housing forming the bulge.

In a second aspect, the present invention provides the use of a housing for a cell seeding chamber in the production of a cell seeding chamber according to the first aspect, the housing having a variable width and a length, an open end to accept a cap comprising an opening for a suction rod insertable into the housing, one or more lateral fluid ports, and a perforated or porous mandrel that can be inserted into the housing and positioned over the suction rod, and a base, wherein the housing has a bulge positioned between about 30% and 60% of the length of the housing forming the bulge.

In a third aspect, the present invention provides the use of a cap for a cell seeding chamber in the production of a cell seeding chamber according the first aspect: the cap comprising an opening for a suction rod insertable into the housing, and one or more lateral fluid ports.

The cell seeding chamber of the first aspect of the invention, which is also referred to herein as the "flip chamber" comprises a housing that has a variable width along its length and wherein the housing has a bulge positioned between about 30% and 60% of the length of the housing forming the bulge. The flip chamber may be operated by flipping the seeding chamber about half-way (180°) during the seeding.

In an alternative "capacitor" chamber design as described herein, although not claimed, the capacitor chamber typically has a uniform width along its length and a narrow gap between the scaffold and the chamber wall.

The cell seeding chamber of the present invention includes a housing, a cap with one, and a base, as further defined above with respect of the first aspect of the present invention. The cap has an opening for a suction rod insertable into the housing, and one or more lateral fluid ports, and for securing a perforated or porous mandrel that can be positioned over the suction rod.

The cap may include one or more lateral tubes, each connecting to a lateral port. The lateral tubes typically include a lateral inlet tube connecting to a lateral inlet port, a lateral outlet tube connecting to a lateral outlet port, and a lateral went tube connected to a lateral vent port. The cap typically has a superior smooth surface and an inferior threaded surface. The threaded surface secures the cap onto the housing. The top portion of the cap is generally flat, and the cap may serve as a base if the chamber is inverted.

The mandrel is typically a perforated, porous mandrel. Alternatively or additionally, the mandrel may have protrusions of any suitable arrangements, such as axially arranged protrusions running in parallel to one another, spiraling down, arranged in a diamond pattern, in circles, in zig-zags, and/or waves.

In an embodiment of the cell seeding chamber of the first aspect of the present invention, or the use of the second and/or third aspect of the present invention, the mandrel is a porous mandrel and the cell seeding chamber has a suction rod inserted therein.

In an embodiment of the cell seeding chamber of the first aspect of the present invention, or the use of the second and/or third aspect of the present invention the cell seeding chamber further comprises a graft or scaffold over the mandrel of the seeding chamber.

The cell seeding chamber may have a gap between about 1 mm and about 30 mm, more preferably between about 3 mm and 20 mm, between the suction rod or the mandrel and the housing for the flip chamber. The housing may have a region with the greatest width positioned between about 30% and 60% of the length of the housing. For example, the region with the greatest width may be positioned at about 50% (half way) along the length of the housing. The housing typically has a threaded portion for receiving the threaded surface of the cap. The 'Flip' seeding chamber eliminates the variability of the distribution of MNCs on the scaffold. The central premise behind the device is to vary the cross section of the seeding chamber along its length. The shape of the chamber resembles that of the inverse of an hour glass, with a bulge (greatest cross section) approximately 40% of the way down the height of the device. Both the top and the bottom of the device narrow to accommodate the mandrel with the scaffold mounted to it, along with a very minimal volume for part clearance and excess liquid. The effect of the change in cross section is that it effectively slows the speed at which the MNCs are drawn through the least seeded portions of the scaffold.

In an embodiment of the cell seeding chamber of the first aspect of the present invention, or the use of the second and/or third aspect of the present invention, the cell seeding chamber has a lateral vent port, and/or comprises a scaffold between the mandrel and the housing.

Also provided are methods for uniformly seeding a graft or scaffold with cells. In a fourth aspect, the present invention provides a method includes the steps of: a) connecting a seeding chamber of the first aspect of the present invention to a closed disposable seeding system containing a vessel with fluid having blood or enriched cells, b) inserting a graft or scaffold over the mandrel of the seeding chamber, c) filling the seeding chamber with the fluid, d) applying negative pressure to a lower mandrel outlet and emptying the chamber to about half way, e) inverting the chamber, and f) applying negative pressure and emptying the chamber through an upper outlet port.

Typically, the seeding chamber is connected to the closed disposable seeding system via the lateral inlet port and the lateral outlet port. The negative pressure may be provided by a syringe, a pump, or a vacuum source. Typically, the graft or the scaffold is uniformly seeded using a minimal volume of a cell-containing fluid, such as a volume between about 10 ml and 200 ml, preferably between 10 ml and 150 ml, of blood, bone marrow aspirate, or mononuclear cell (MNC)-enriched fluid.

## BRIEF DESCRIPTION OF THE DRAWINGS

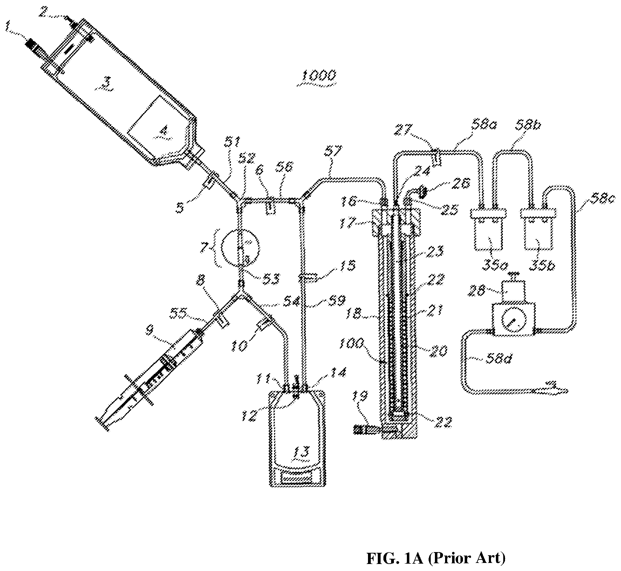

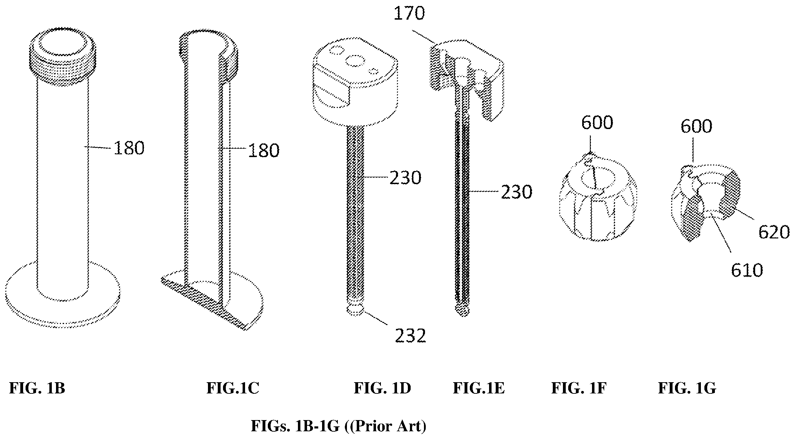

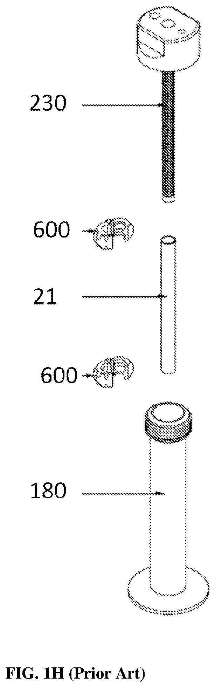

Fig. 1A is a diagram illustrating an exemplary closed system (1000) for seeding and/or testing cells and/or grafts, e.g., tissue grafts as described in US Patent No. 9,090,863 and US Application Publication No. US 2018/0353649. Fig. 1B is a drawing illustrating a perspective view of an advanced seeding chamber (180) that can be used in place of the seeding chamber (18) shown in Fig 1A. Fig. 1C is a drawing illustrating a cross-sectional perspective view of the seeding chamber (180) that can be used in place of the seeding chamber (18) shown in Fig 1A. Fig. 1D is a drawing illustrating a perspective view of the suction rod (230) containing an integral threaded top (170) that can be used in place of the seeding chamber (18) shown in Fig 1A. Fig. 1E is a drawing illustrating a cross-sectional perspective view of the suction rod (230) containing an integral threaded top (170), and also containing an indent at the base (232) configured to receive a correspondingly shaped clip (600). A similar indent is provided at the top of the suction rod. Fig. 1F is a drawing illustrating a perspective view of a clip (600), that can be used in place of the O-ring (22) within the seeding chamber (100) shown in Fig. 1A. The clip (600) is typically hollow, having a solid wall (620), surrounding a hollow center (610). Optionally the wall (620) contains a hinge to facilitate opening and closing of the clip (600). Fig. 1G is a drawing illustrating a cross-sectional perspective view of a clip (600) within the seeding chamber (100) shown in Fig. 1A. Fig. 1H is an exploded view illustrating the assembly of the different components of a seeding chamber assembly (200), that can be used in place of the seeding chamber assembly (100) shown in Fig. 1A, including the suction rod (230), scaffold (20), scaffold clips (600) and seeding chamber (180).

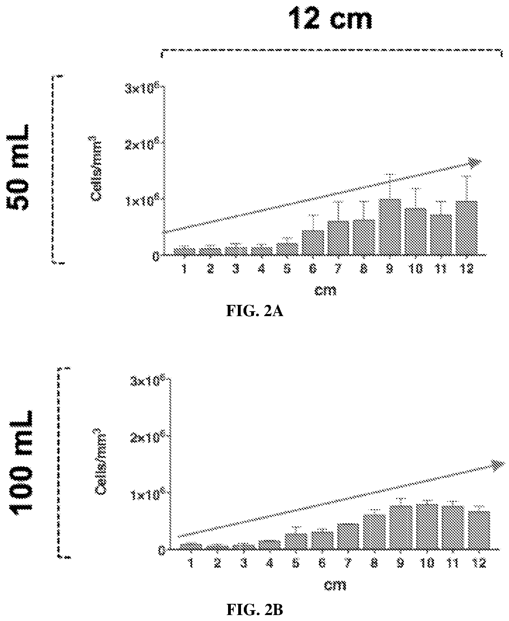

Figures 2A and 2B are bar graphs showing the seeding gradient of cells (Cells/mm$^3$ because the scaffolds are 1 mm thick, the values are presented as Cells/mm$^3$) along the length (cm) of the scaffold using 50 mL or 100 mL of bone marrow with standard closed, disposable seeding system of the prior art (US Patent No. 9,090,863 B2 and US Application Publication No. US 2018/0353649).

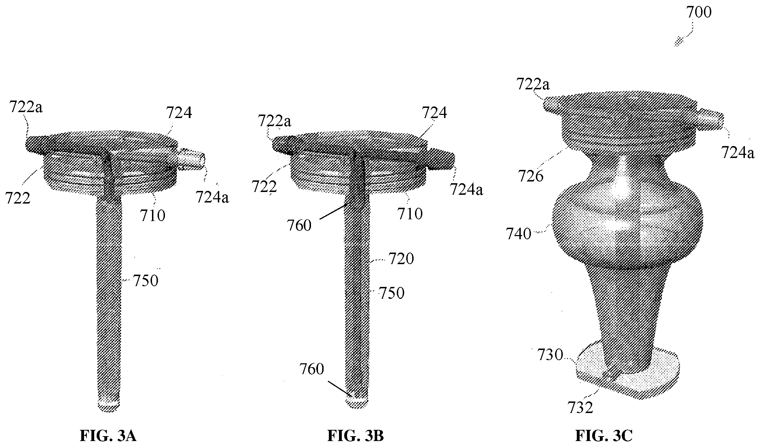

Figures 3A-3E are diagrams showing the assembly and use of a seeding chamber with a flip design - "flip" seeding chamber.

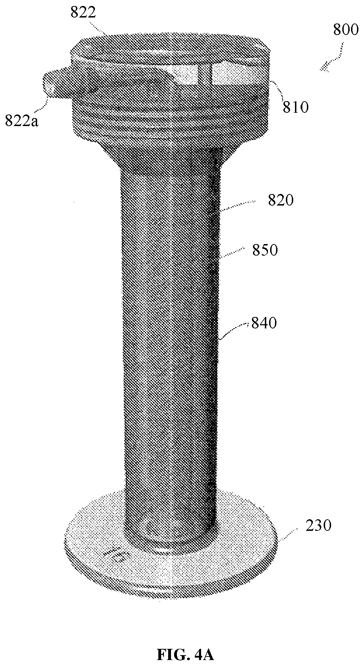

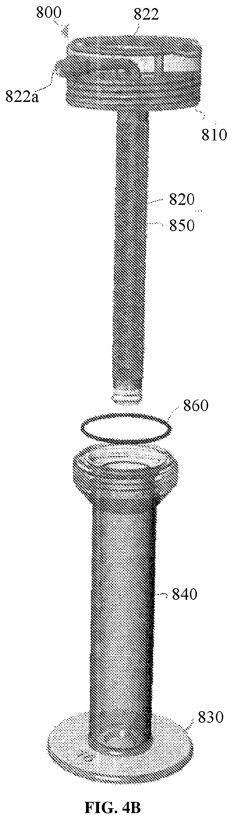

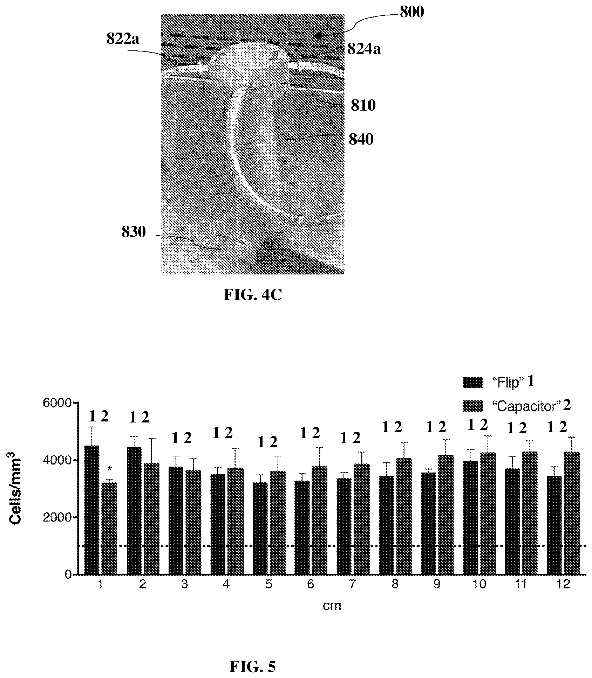

Figures 4A and 4B are diagrams showing the assembly of a seeding chamber with a capacitor design - "capacitor" seeding chamber. Figure 4C is a diagram of the assembled seeding chamber connected to the seeding system, including IV bag, vacuum source, drain, and air bleed lines.

Figure 5 is bar graph showing uniform seeding density (Cells/mm$^3$) along the length (cm) of the scaffold seeded in the flip seeding chamber ("Flip", 1) or capacitor seeding chamber ("Capacitor", 2).

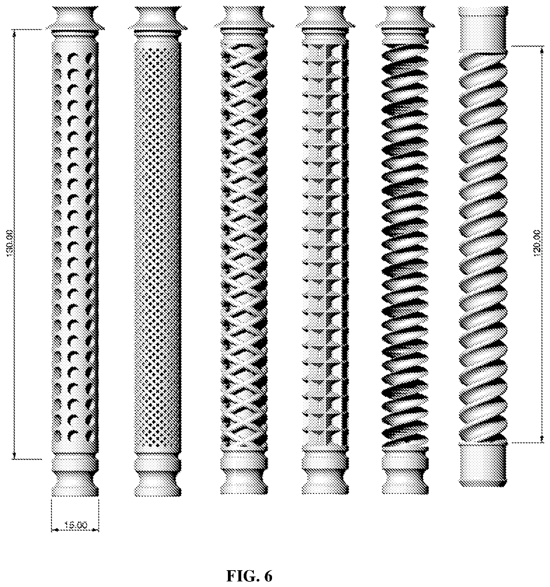

Figure 6 is a diagram showing the different structures of a mandrel for use with the closed disposable seeding system. The dimensions shown are: the total length 130 mm, length of the seeding region 120 mm, width 15 mm.

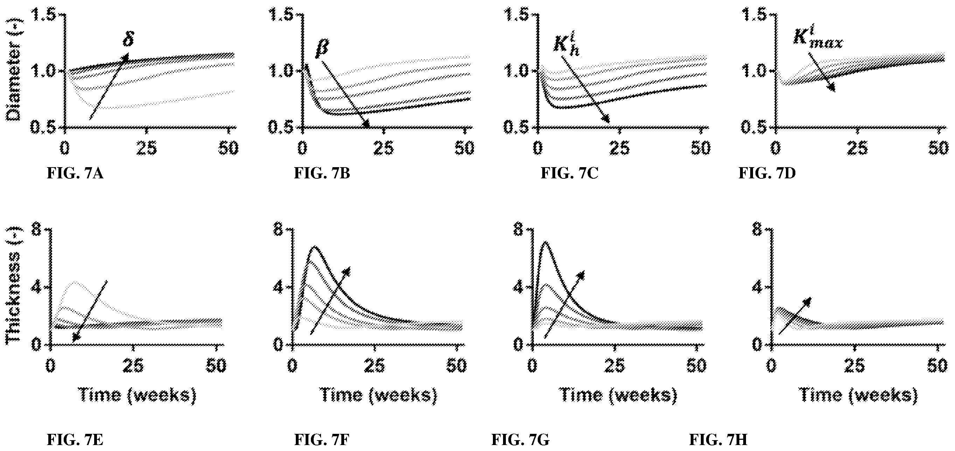

Figures 7A-7H are line graphs showing change in diameter (Figures 7A-7D) or thickness (Figures 7E-7H) over time (weeks) with the change in four key parameters: $\delta$, $\beta$, $K^i_h$, and $K^i_{max}$. Shown are parametric studies for these four key parameters: $\delta$ modulates the onset and duration of the inflammatory response, $\beta$ dictates the shape and skew of the inflammatory timecourse, $K^i_h$ controls the peak rates of inflammatory neotissue production and degradation, and $K^i_{max}$ scales inflammatory effects on neotissue degradation.

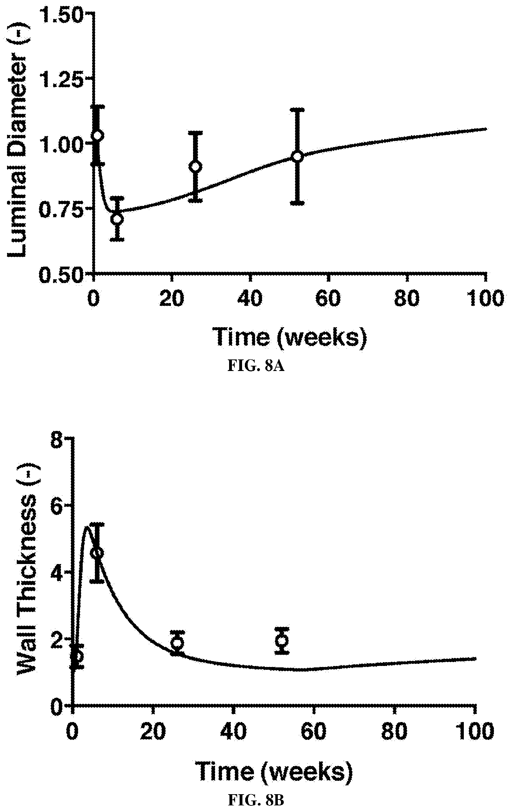

Figures 8A and 8B are line graphs showing plotted model simulations (solid lines) for luminal diameter (Figure 8A) or wall thickness (Figure 8B) over time (weeks) of the TEVG fit well with actual intravascular ultrasound experimental measurements of hydraulic diameter acquired over the 1 year study period (symbols $\pm$ SD).

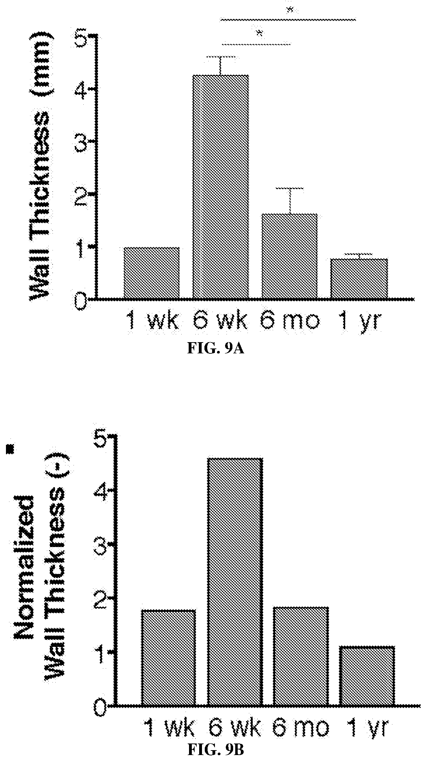

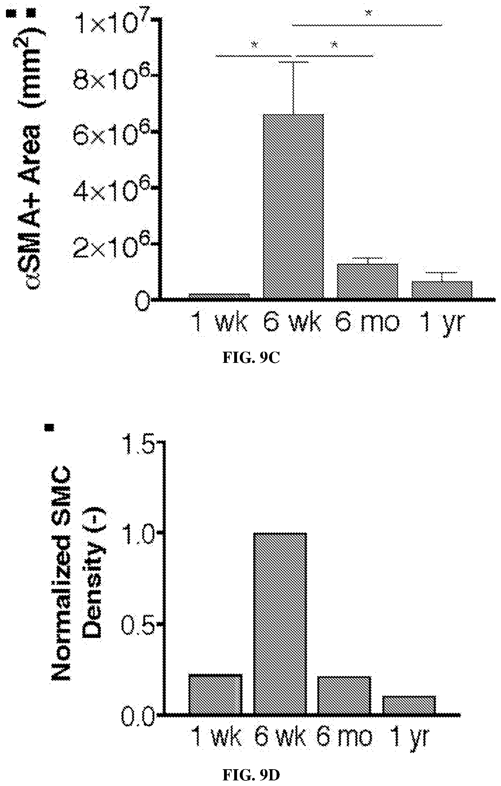

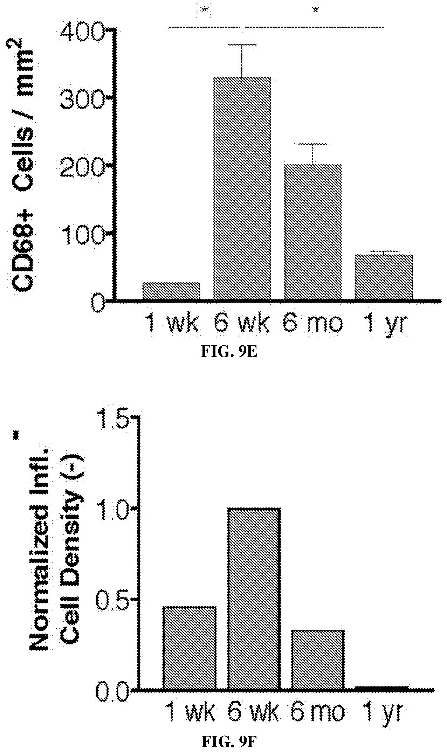

Figures 9A-9F are bar graphs showing stenosis arises from excessive inflammation-driven neotissue formation. Quantitative histological analysis demonstrates that wall thickness (Figure 9A) is greatest 6 weeks after implantation, corresponding with model simulations (Figure 9B) of TEVG wall thickness over time (one-way ANOVA with Tukey's multiple comparisons test: a= 0.05, *$p$ <0.05). Figure 9C shows quantifications of aSMA+ area within the vascular neotissue over time is consistent with model predictions (Figure 9D) of an increased smooth muscle cell density at 6 weeks (one-way ANOVA with Tukey's multiple comparisons test: a= 0.05, *$p$ < 0.05). Similarly, CD68+ macrophage staining and quantification (Figure 9E) confirm that significant inflammation corresponds with the development of TEVG stenosis (one-way ANOVA with Tukey's multiple comparisons test: a= 0.05, *$p$ < 0.05). This observation also closely matched computational model predictions of inflammatory cell density (Figure 9F). Plotted data represent means $\pm$SEM.

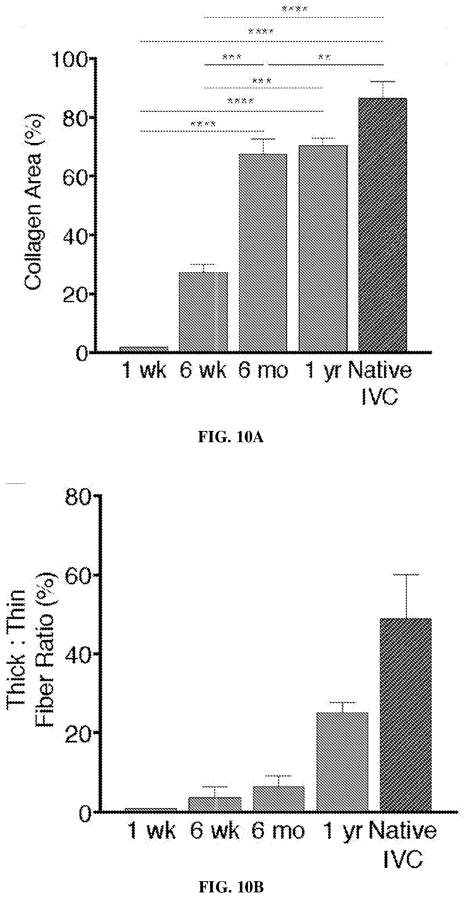

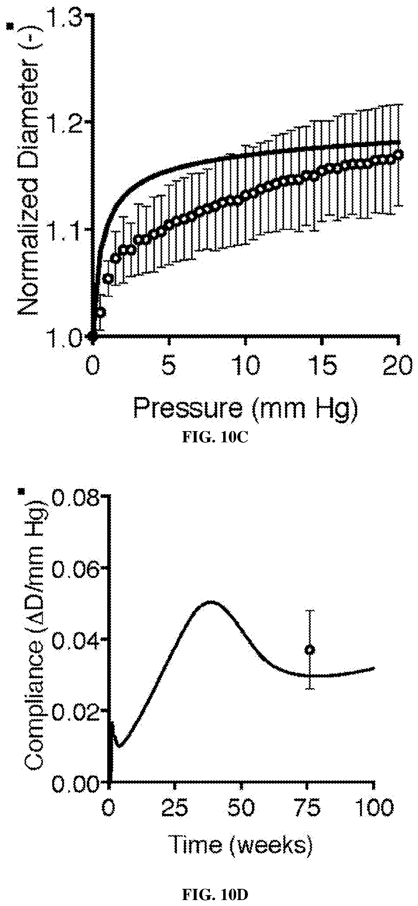

Figures 10A-10D are graphs showing changes in biomechanical properties of the implanted grafts accurately follow computational model predictions. Figure 10A is a bar graph showing quantification of total collagen area fraction (%) from the Picro-Sirius Red stain over time compared to the native IVC (last bar) (one-way ANOVA with Tukey's multiple comparisons test: a= 0.05, **p < 0.005, ***p < 0.0005, ****p < 0.0001, plot represents means ± SEM). Figure 10B is a bar graph showing the ratio (%) of thick to thin collagen fibers in the TEVG over time compared to the native IVC (last bar) (means± SEM). Figure 10C is a graph showing the experimental measurements of the mechanical behavior of the TEVG (Normalized Diameter) in response to *in vitro* pressurization after 1.5 years of development *in vivo* (symbols ± SD) were consistent with predicted values from the computational model (solid line). Figure 10D shows the predicted graft compliance from 2 to 3 mmHg through 2 years of simulated neovessel development (solid line) with reasonable agreement to the experimentally measured compliance at 18 months (symbols ± SD).

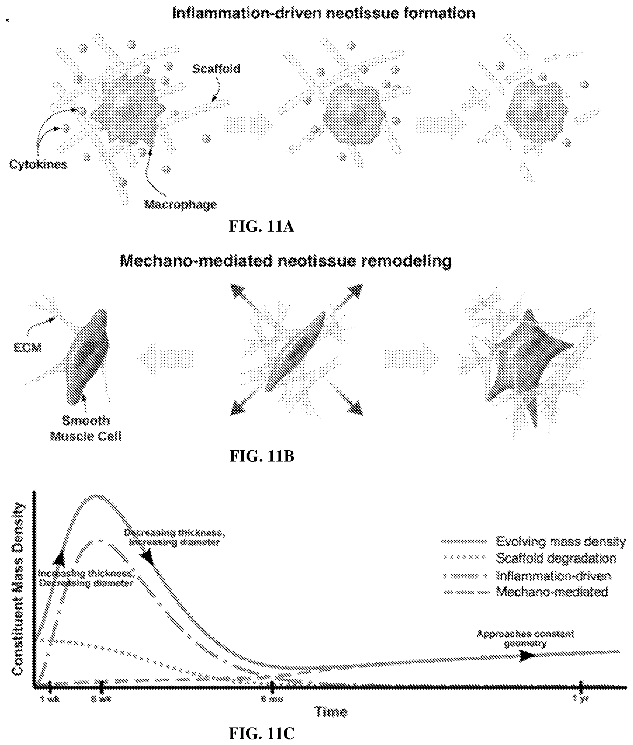

Figures 11A and 11B are diagrams showing implantation of the scaffold induces a foreign body reaction and mechano-mediated neotissue remodeling. Figure 11C is a graph of the computational model showing the interplay of scaffold degradation on inflammation-driven and mechano-mediated neotissue formation. Factors driving the evolving mass of constituents in the computational model of TEVG development include the loss of polymeric scaffold (•••••••) and the gain of inflammation-driven (- • -) and mechanobiologically-mediated (- - -) neotissue. The sum of each of these constituents (-) determines the mass density of the neotissue.

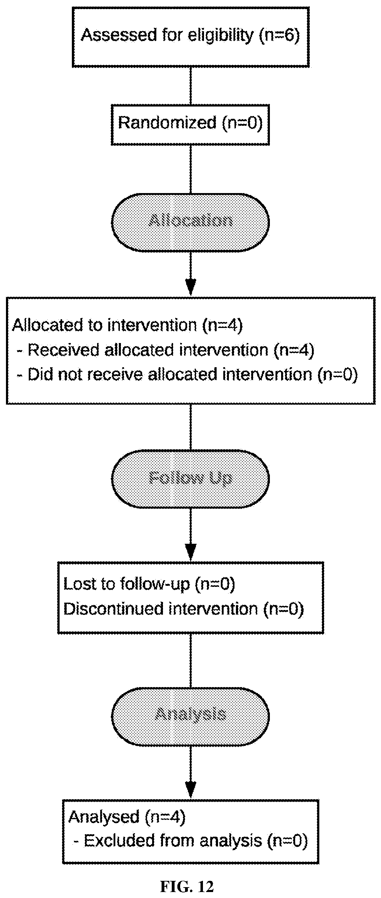

Figure 12 is a flow diagram showing the pilot study investigating the clinical use of tissue engineered vascular grafts in congenital heart surgery.

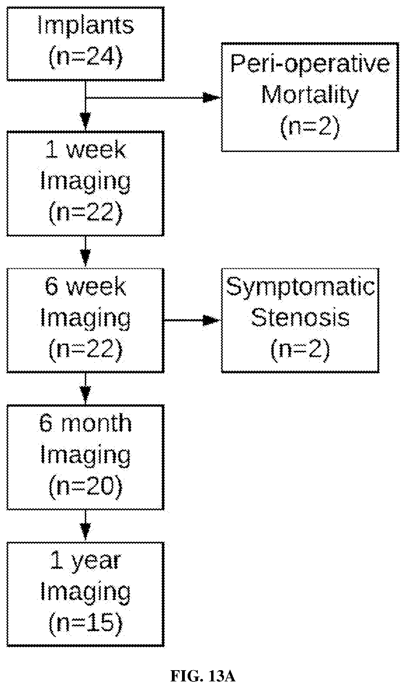

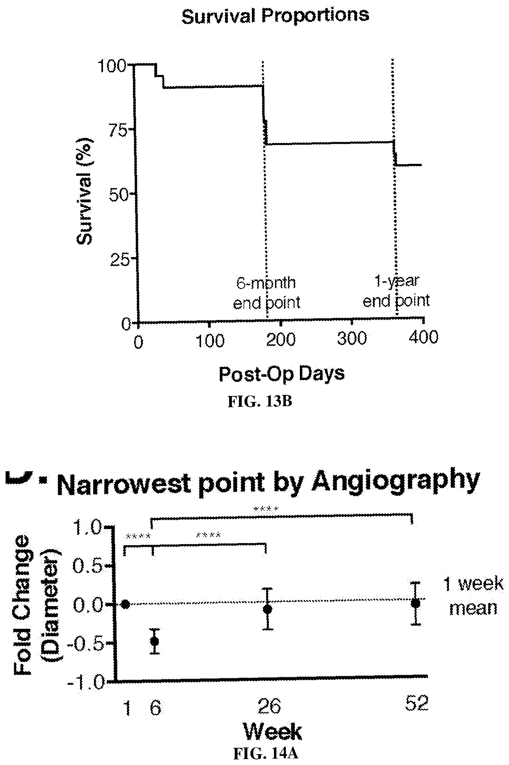

Figure 13A is a flow diagram showing study design and number of animals analyzed at each time point for the ovine study. Figure 13B is a survival curve for the ovine study showing survival (%) over post-operative days.

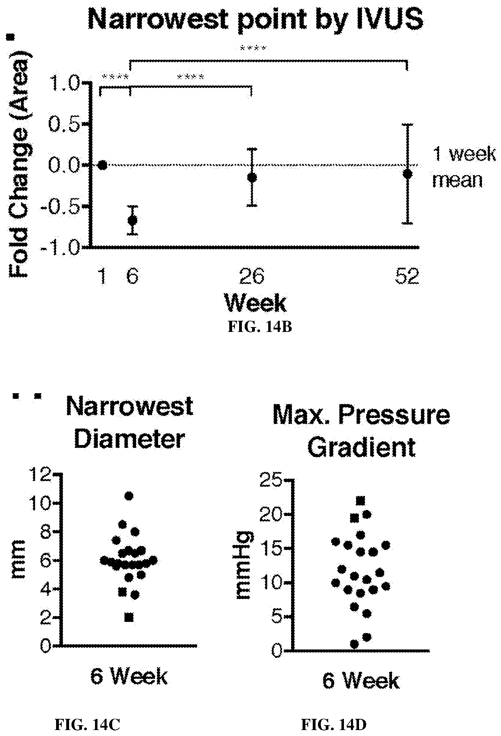

Figures 14A-14D are graphs showing changes in different parameters of the implanted TEVG in the ovine study. Angiogram and intravascular ultrasound of the TEVG in the ovine model were used. Data were obtained from representative serial angiographic and IVUS images of one ovine TEVG at 1 week, 6 weeks, 6 months, and 1 year after implantation demonstrating the development of critical stenosis at 6 weeks followed by remodeling into a patent neovessel by 6 months. Plots of relative luminal diameter (Fold change, diameter, Figure 14A) and area change ((Fold change, area, Figure 14B), as fold change relative to 1 week, of the TEVG from serial angiogram and intravascular ultrasound studies over 1 year are presented demonstrating that narrowing of the TEVG spontaneously reverses by 6 months (one-way ANOVA with Tukey's multiple comparisons test: a= 0.05, ****p < 0.0001, means ± SD). The 1 week mean values are identified by dashed horizontal lines. Figures 14C and 14D are graphs showing six-week diameter (Figure 14C) and pressure (Figure 14D) gradient measurements from each animal in the study cohort. Two animals experienced asymptomatic ascites and two animals experienced symptomatic ascites requiring euthanasia before the study end point (squares).

## DETAILED DESCRIPTION

### I. Definitions

The term "Tissue-Engineered Vascular Graft (TEVG)" refers to a vascular graft or scaffold that is designed for insertion into the body for use in the repair or augmentation of one or more vessels, such as arteries and veins.

The term "biocompatible" refers to a material that the body generally accepts without a major immune response, which is capable of implantation in biological systems, for example, tissue implantation, without causing excessive fibrosis or rejection reactions.

The term "biodegradable" refers to the ability of a substance or material to break down when exposed to water, enzymes or in an *in vivo* environment.

As used herein, "stenosis" refers to a reduction in lumen diameter of 25% or more relative to lumen diameter of scaffold at implantation.

The term "mandrel" refers to a cylindrical device or tube, *e.g.,* a metal bar, that serves as a core around which material, *e.g.*, a matrix scaffold for seeding and growing cells, may be cast, molded, forged, bent, or otherwise shaped. The mandrel is typically open on at least one end. The mandrel may also contain holes or perforations along its longitudinal axis (*i.e.,* along its length).

As used herein, the term "porous" refers to having one or more openings, pores, perforations or holes that may be filled or perfused by a liquid and/or a gas, or that allows for the flow of a liquid and/or gas therethrough.

As used herein, the term "spontaneous" in the context of reversal of stenosis refers to an action occurring without an additional invasive or non-invasive procedure, such as without a surgical intervention or a surgical correction.

As used herein, the term "reversal of stenosis" refers to reduction of stenosis by at least about 70%, 75%, 80%, 85%, 90%, 95% or by 100% relative to a an implanted graft without seeded cells and stenosed.

As used herein, the term "substantially" refers to a measure of about 80%, about 85%, about 90%, about 95%, or about 98%.

### II. Systems for Seeding TEVGs

## A. Closed Disposable Seeding Systems

Systems and methods thereof for seeding TEVGs with cells are known in the art. An exemplary system is described in U.S. Patent No. 9,090,863 and in US 2018/0353649 (**Figure 1A, prior art**). The device illustrated in Figure 1A is a closed system (1000) for seeding, culturing, storing, shipping and/or testing cells and/or grafts, e.g., tissue grafts. The operation, structure, and results of prior seeding systems are discussed and illustrated further in U.S. Patent No. 9,090,863and in Tissue Engineering Part C: Methods. (1):88-93 (2014).

Typically, systems for seeding of cells into tissue engineered vascular grafts include means for creating a vacuum that is connected to a patient for extracting biological material from the patient, into a chamber or scaffold. The scaffold acts as an incubator, allowing at least a portion of the biological material to contact and interact with the scaffold. The scaffold is in fluid communication with a filter/switch combination that allows selectable fluids to transfer from the biological material, back into the patient. An exemplary biological materials that are transferred back into the patient include serum, red bloods, platelets, white blood cells and combinations. Extracting selected biological materials from the fluid that contacts the scaffold reduces the time required for seeding the scaffold with a desired cell types, and increases healing rates in the patient.

The component parts of an exemplary system, described in c, are illustrated in **Figures 1A-1H (prior art).** In general, the components include:

port (1) or port (2)
cellular isolate fluid container (3)
valve (5)
fluid line (51, 52, 53, 54, 55, 56, 57, 58a-58d, and 59)
pre-filter element (4)
flow channel (7)
container (13)
inlet port (11)
outlet port (14)
valve (10)
seeding container (18)
container (9)
valve (8)
threaded cap (17)
inlet port (16)
outlet port (24)
sampling port (19)
vent port (26)
fluid containers 35a and 35b
port (25)
vacuum source, e.g., a pump, and a regulator (28)
seeding assembly (100)
porous tube (20)
scaffold (21)
clips (60)

The components are interconnected and used as described in U.S. Patent No. 9,090,863 and US 2018/0353649.

For example, the system includes a vessel for containing a cellular isolate, e.g., a cellular isolate fluid, such as a container (3). Exemplary containers include a media bag or any flexible or rigid container capable of being sterilized and/or that is hermetically sealed (e.g., Gibco-BFL 1 L media bag). In certain embodiments, the cellular isolate fluid container (3) is formed from of a biocompatible, rigid material capable of being sterilized, such as TEFLON®, polycarbonate, polyvinyl chloride (PVC), or stainless steel. The container (3) can have any suitable volume for containing the cellular isolate fluid, typically less than 250 ml. In a preferred embodiment, the container (3) has at least one port adapted for the sterile filling and/or dispensing of a fluid, for example, a bone marrow aspirate. For example, a bone marrow aspirate (e.g., 5 ml/kg body weight) is aseptically collected and passed, e.g., injected, into container (3) via port (1) or port (2).

Typically, the container (3) has at least one inlet and one outlet. In some embodiments, the container (3) includes a port having one or more valves to allow for the one-way flow of a fluid or gas. For example, using the embodiment shown in **Fig. 1A** for reference, port 1 can include and/or take the form of a swabable valve, such as a needleless access port. The valve can be connected to container (3), and/or can be associated with a fluid line communicating with the container (3), using any suitable coupling or fastening means which is known to those in the art, e.g., a clamp, screw or luer connector, pressure fitting, friction fitting, or coupling. In accordance with the embodiment of the system (1000) illustrated in **Fig. 1A,** valve (5) is associated with a scaffold, *e.g.,* a fluid line (51), communicating with the container (3). Optionally, the container (3) includes a pre-filter element (4), *e.g.*, including, for example, a screen or woven element having openings or a pore structure in the range of, for example, about 40 to about 150 microns. Such pre-filter element could be used to remove undesirable material such as, *e.g.,* bone chips, clots, and/or fat deposits, as the fluid passes from the container.

Examples of fluid which may be used in the system include, but are not limited to, sterilizing fluid, contrast media fluid, biological fluid, fluid containing cells, blood, serum, bone marrow aspirate, or fluid containing a culture medium. It is to be understood that during testing, seeding, and culturing in a preferred embodiment, the fluid may be kept at human body temperature, and may be composed of a fluid which approximates the viscosity of human blood. One illustrative example of a solution which approximates the viscosity of blood is saline with glycerol.

The fluid in container (3) is passed from the container through fluid line (51) of **Fig. 1A.** In a preferred embodiment, the fluid is directed away from container (3) by a vacuum. In other embodiments, the system incorporates the use of a fluid pump. Fluid pumps are available from multiple commercial sources (*e.g.*, Masterflex L/S Digital Drive peristaltic pump manufactured by Cole-Palmer).

Fluid line (51), as well as all other fluid lines in the system (*e.g.*, lines 52, 53, 54, 55, 56, 57, 58a-58d, and 59), may be made of any type of medical grade, sterilizable, durable tubing suitable for transporting the fluid or gas in use. For example, the fluid line can be flexible or rigid plastic.

The system also includes a flow channel (7) including at least one inlet, at least one outlet and at least one filter including at least one filter medium (*e.g.*, disposed in a filter housing) between. In a preferred embodiment, the filter is disposed at an angle that is approximately perpendicular to the direction of flow through the flow channel (7), although in some embodiments, the filter can be disposed at an angle approximately parallel to the direction of flow, *e.g.,* tangential flow filtration. In preferred embodiments, the filter is adapted to allow flow through in at least two directions, for example, where the first and second directions are approximately opposite, *e.g.,* wherein a fluid can be passed in a first direction from the upstream surface of the filter through the downstream surface, and a fluid can be passed in a second direction from the downstream surface of the filter though the upstream surface. In an example of this embodiment, a cellular isolate fluid is passed in a first direction through a filter having a suitable pore size (or mesh size), wherein the filter medium is at an angle that is approximately perpendicular to the direction of flow such that the filter retains cells and/or biological material that is too large to pass through the filter. A second fluid is subsequently passed in a second direction through the filter which can wash the retained cells and/or biological material off of the filter medium. Filters that can be employed for use in the flow channel are well known in the art and include, for example, Pall Corporation. In other embodiments, the filter (*e.g.,* at least one filter medium) has a porosity suitable to retain cells, *e.g.,* bone marrow-derived mononuclear cells. In certain embodiments, the filter includes a matrix that is designed to reversibly bind and retain the cells of interest based upon, for example, ligand-receptor interactions. In other embodiments, multiple filters can be assembled in series or in parallel for use in the system as described herein. It is contemplated that the system and method can have any number of desired flowpaths and shutoffs. The liquid or gas can be fed through the system by positive pressure or negative pressure, such as *via* a syringe, pump, or vacuum source.

In certain embodiments, the system includes a means for containing a collection fluid. In certain embodiments the means is a container (13), such as a media bag or any flexible or rigid container capable of being sterilized and/or that is hermetically sealed (*e.g.,* GIBCO-BFL 1 L media bag). In certain embodiments, collection container (13) is formed from a biocompatible, rigid material capable of being sterilized such as Teflon, polycarbonate, acrylic, PVC, or stainless steel. Container (13) can have any suitable volume for containing the collection fluid.

In a preferred embodiment, the container (13) has at least one port adapted for the sterile filling and/or dispensing of a fluid, for example, a bone marrow aspirate filtrate or flow through. For example, a bone marrow aspirate (*e.g.,* 5 cc/kg body weight) is aseptically collected and passed, *e.g.,* injected, into container (3) and subsequently passed (*e.g.*, through optional pre-filter (4) and *via* fluid flow lines (51) and (52) through flow channel (7) including a filter. The filter retains cells and/or the biological material of interest, allowing the filtrate to flow through fluid lines (53) and (54) and inlet port (11) into container (13). In another preferred embodiment, the container (13) has at least one flow port, *e.g.,* a bi-directional flow port; typically, however, the container (13) has at least two ports. In certain embodiments, container (13) includes an inlet port and/or an outlet port.

In the embodiment illustrated in **Fig. 1A,** container (13) includes an inlet port (11) and an outlet port (14). In still another embodiment, the container (13) includes a port having one or more valves to allow for the one-way flow of a fluid or gas. The valve can be connected to container (13) and/or associated with a fluid line communicating with container (13) using any suitable coupling or fastening means which is known to those in the art, *e.g.,* a clamp, screw or luer connector, pressure fitting, friction fitting, or the like. In accordance with the embodiment illustrated in **Fig. 1A,** the system (1000) includes a valve (10) associated with the fluid line (54).

In certain embodiments (*e.g.,* after elution fluid is passed through the flow channel (7) and cells are passed into seeding container (18) as noted in more detail below), the collection fluid or filtrate contained in container (13) is passed through fluid lines (59) and (57) into seeding container (18) (in **Fig. 1A**).

In a preferred embodiment, the fluid is directed away from container (13) by a vacuum. In other embodiments, a fluid pump is used (*e.g.,* Masterflex L/S Digital Drive peristaltic pump manufactured by Cole-Palmer, although one skilled in the art could select from a variety of commercially available pumps).

In certain embodiments, the system includes a means for containing an elution fluid. In certain embodiments, the means is a container (9), such as a media bag, syringe, or any flexible or rigid container capable of being sterilized and/or that is hermetically sealed (*e.g.,* a Gibco-BFL 1 L media bag).

In accordance with the embodiment illustrated in Fig. 1A, the system (1000) includes a valve (8) associated with the fluid line (55). The valve can be connected to container (9) and/or associated with the fluid line using any suitable coupling or fastening means which is known to those in the art, e.g., a clamp, screw or Luer connector, pressure fitting, friction fitting, or the like. The elution or wash fluid is passed through valve (8) and fluid line (53), flow channel (7), fluid lines (52), (56), and (57), into seeding container (18) in Fig. 1A.

In one embodiment, container (9) is a syringe filled with a sterile elution or wash fluid. The elution or wash fluid is passed through flow channel 7 and into seeding container (18) through valve (8), fluid lines 53, 52, 56 and 57 in **Fig. 1A.** In this embodiment, the fluid is directed away from container 9 due to pressure exerted on the fluid by depressing the syringe plunger, for example, manually or via a mechanical and/or electrical device. Alternatively, for example, the container (9) can be a flexible container that can be compressed. However, as one of skill in the art would readily appreciate, a fluid pump could also be used (e.g., MASTERFLEX L/S DIGITAL DRIVE peristaltic pump manufactured by COLE-PALMER, although one skilled in the art could select from a variety of commercially available pumps).

In certain embodiments, the system includes a means for containing a cell seeding assembly. In certain embodiments, the means is a seeding container (18), such as a media bag or any flexible or rigid container capable of being sterilized and/or that is hermetically sealed. For example, a GIBCO-BFL 1 L media bag could be used. In certain embodiments, container 18 may be composed of any biocompatible, rigid material capable of being sterilized such as Teflon, polycarbonate, acrylic, PVC, or stainless steel. Seeding container (18) can have any suitable volume.

As illustrated in **Fig. 1A,** the seeding container (18) includes a rigid material containing a main body section including threads, and a threaded cap (17). The container (18) includes at least an inlet and an outlet port (in the embodiments illustrated in **Fig. 1A,** seeding container (18) includes an inlet port (16), an outlet port (24), a sampling port (19) (e.g., for aseptic acquisition of fluid samples from the seeding container to determine, for example, microbial contamination and/or stem cell enumeration), and a vent port (26), wherein the cap 17 (**Fig. 1A**) includes the ports).

The seeding container 18 includes an inlet port 16 and outlet port 24, which allows for the perfusion and/or circulation of fluid into and through the container. Inlet port 16 and outlet port 24 are also used to attach container 18 to fluid lines 57 and 58a, respectively. Fluid line 58a connects seeding container 18 to one or more residual seeded cell fluid containers 35a and 35b, while maintaining a closed system. It is to be understood that although only one seeding container 18 is shown in **Fig. 1A,** a fluid line, e.g., fluid line 57 or 58a, may be branched so as to connect more than one seeding container in parallel to the system.

The means for containing residual seeded cell fluid is at least one residual seeded cell fluid container 35a, 35b, such as a media bag or any flexible or rigid container capable of being sterilized and/or that is hermetically sealed. For example, a Gibco-BFL 1 L media bag could be used. In certain embodiments, containers 35a, 35b may be composed of any biocompatible, rigid material capable of being sterilized, such as TEFLON®, polycarbonate, PVC, or stainless steel.

In a preferred embodiment, fluid is drawn out of container 18 into a residual seeded cell fluid container 35a via port 25 and fluid line 58a through the use of vacuum assembly having a vacuum source, e.g., a pump, and a regulator 28, wherein the negative pressure from the pump is conveyed through fluid lines connected to residual seeded cell fluid container 35a, 35b and seeding container 18.

In certain embodiments, seeding container (18) houses a seeding assembly (100) containing a porous tube (20) and a scaffold (21), e.g., cell or tissue scaffold or graft, such as a vascular graft scaffolding. The porous tube (20) may include any suitable rigid material, such as TEFLON, PVC, polycarbonate, plastic, metal, e.g., stainless steel, which may be made fluid permeable. One or more retaining elements such as clips (60), O-rings, or grommets may also be placed on tube, e.g., at both ends of scaffolding (21), to hold the scaffolding in place on the tube during seeding, culturing, storing, shipping, or treatment.

In certain embodiments, the system is disposable. The closed disposable system allows for a procedure for the construction of tissue engineered graft, *e.g.,* a vascular graft, that can be performed rapidly while achieving similar seeding efficiency as compared to previously described methods (Matsumura, et al., Biomaterials 2003; 24:2303-8; and FDA IDE 14127). In addition, the use of the system allows one to construct the tissue engineered graft, *e.g.,* vascular graft, at the point of care (*i.e.,* in the operating room precluding the need for scaffold transport.

As described herein, it was discovered during clinical trials that scaffolds and grafts having non-uniform cell seeding density along the length of the scaffold or graft lead to an increased incidence of graft stenosis following implantation. Improved seeding chambers for use in a closed disposable seeding system have been developed which provide a uniform density of cells seeded along the length of the scaffold or graft. The cell seeding chamber, termed "flip" chamber and "capacitor" chamber, have a cap with lateral ports. The present invention focusses on the "flip" chamber development. Thus, the seeding system uses a seeding chamber **700** (flip seeding chamber), rather than an alternative design discussed herein, in which the seeding system uses a seeding chamber **800** (capacitor seeding chamber) instead of the seeding container **18.**

## 1. Seeding Chambers

The seeding chambers, for either the "flip" chamber design of the present invention or the alternative unclaimed "capacitor" chamber design as described herein, generally include a housing having a width and a length, a cap with one or more lateral ports, and a base. The cap typically includes a suction rod insertable into the housing, and a mandrel positioned over the suction rod. Typically, the cap is flat on its upper surface. The cap generally does not include valves, ports, or attachments on the upper surface.

In an exemplary embodiment, the seeding chamber base has a radius of between 10 and 100 mm, for example, between about 35 mm and 80 mm. In an exemplary embodiment, the top of the seeding chamber has a diameter of between 10 and 100 mm, between about 25 mm and 60 mm, for example, 38.1mm. In an exemplary embodiment, the seeding chamber base has a height of between 20 and 1000 mm, between about 120 mm and 200 mm, for example, 180 mm. Typically, the inner diameter of the seeding chamber aperture is between 5 and 90 mm, for example, 25.86 mm. A typical thickness for the wall of the seeding chamber is between 0.5 and 10 mm, for example, approximately 2 mm. When the seeding chamber has a threaded top, the height of the threaded section is typically approximately 10% of the total length of the chamber, for example, 20.55 mm. The mandrel, seeding chamber, and one or more scaffold clips are typically sized corresponding to the desired size of the vascular graft, seeding chamber, and are sized to fit together within the seeding chamber.

### a. Flip Seeding Chamber

The 'Flip' seeding chamber design of the first aspect of the present invention comprises a housing that has a variable cross section diameter along its length, and a bulge positioned between about 30% and 60% of the length of the housing forming the bulge.

Typically, the shape of the chamber resembles that of the inverse of an hour glass, with greatest cross section approximately 30% to 60%, preferably about 40%, of the way down the height of the device. Both the top and the bottom of the device narrow to accommodate the mandrel with the scaffold mounted to it, along with a very minimal volume for part clearance and excess liquid. Typically, the cross section diameter of the flip chamber at its narrowest portions is between about 22 and 25 mm, inclusive. Typically, the cross section diameter of the flip chamber at its widest portion is between about 60 and 80 mm, inclusive.

The effect of the change in cross section diameter is that it effectively slows the speed at which the MNCs are drawn through the least seeded portions of the scaffold.

In addition, the mandrel for this design has two vacuum nubs - one to draw negative pressure in the upright orientation, and one to draw negative pressure in the inverted orientation.

An exemplary flip seeding chamber is presented in Figures 3A-3E. The flip seeding chamber **700** includes a cap **710,** a housing **740,** and a base **730.** The cap includes lateral ports, such as inlet port **722a** connected to an inlet tube **722,** and an outlet port **724a** connected to the outlet tube **724.** The cap **710** has a flat upper surface. The cap **710** may have an upper surface of any shape. Typically, the upper surface of the cap is free of ports, tubes, or vents.

The housing **740** has a variable cross-sectional diameter along its length. The upper 10% of the housing includes threats to receive the threaded portion of the cap **710.** The bottom section of housing **740** is connected to the base **730.** The housing **740** may include a lower mandrel outlets **732** and **734.** O-ring grooves **760** may also be present for positioning of o-rings to seal scaffold to mandrel.

The cap **710** is connected to the suction rod **720.** A porous mandrel **750** is attached to the suction rod **720.** The attachment may be by any means, including clamp, screw or luer connector, pressure fitting, friction fitting, or coupling.

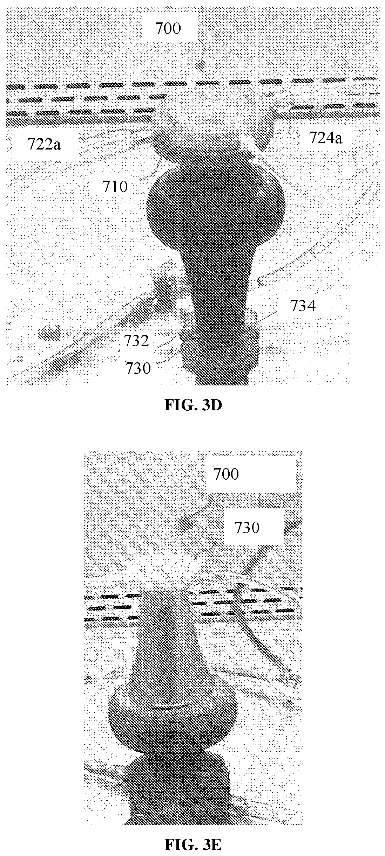

Typically, the flip seeding chamber is configured to stably hold in the vertical position when positioned on its base, or when positioned on its cap. This is shown in Figures 3D and 3E.

### b. Capacitor Seeding Chamber

The alternative capacitor seeding chamber as described herein is typically a hollow tube having a consistent cross-sectional diameter along the length of the tube. Typically, the cross section diameter of the capacitor chamber is between about 10 and 20 mm, inclusive. The capacitor seeding chamber is very narrow as compared to the assembled mandrel/scaffold assembly. It is designed to hold a minimum volume of fluid. The minimum and the maximum gap between the scaffold and the wall of the chamber may be about 1 mm and about 5 mm.

An exemplary capacitor seeding chamber is presented in Figures 4A-4C. The capacitor seeding chamber **800** includes a cap **810,** a housing **840,** and a base **830.** The cap includes lateral ports, such as inlet port **822a** connected to an inlet tube **822,** and an outlet port **824a** connected to the outlet tube **824.** The cap **810** has a flat upper surface. The cap **810** may have an upper surface of any shape. Typically, the upper surface of the cap is free of ports, tubes, or vents.

The housing **840** has a constant cross-sectional diameter along its length. The upper 10% of the housing includes threads to receive the threaded portion of the cap **810.** An O-ring **860** may be positioned between the cap **810** and the housing **840.** The bottom section of housing **840** is connected to the base **830.**

The cap **810** is connected to the suction rod **820.** A porous mandrel **850** is attached to the suction rod **820.** The attachment may be by any means, including clamp, screw or luer connector, pressure fitting, friction fitting, coupling or the like.

## 2. Mandrel

Mandrels are typically sized to fit over suction rod. The mandrels may be secured to the suction rod or to the cap with any suitable attachments. Exemplary attachments include clips, hooks, and slots accepting mandrel width. The mandrel may be attached to the suction rod by molding or gluing.

The mandrel may have any shape suitable to receive a vascular graft or scaffold. The mandrel is typically a perforated, porous mandrel. Alternatively or additionally, the mandrel may have protrusions of any suitable arrangements, such as axially arranged protrusions running in parallel to one another, spiraling down, arranged in a diamond pattern, in circles, in zig-zags, and/or waves.

Exemplary dimensions for the mandrel are provided in Figure 6. In an exemplary embodiment, the length of the mandrel that will fit within a 180 mm long seeding chamber is between about 120 mm and 140 mm, such as about 128 mm.

## B. Fluid Volume, Cell Density, and Seeding Time

Seeding of the graft or the scaffold typically includes contacting the graft or scaffold with fluid containing cells.

Accordingly, a fourth aspect of the present invention provides a method for seeding a graft or scaffold with cells, the method comprising: a) connecting the seeding chamber of the first aspect of the present invention to a closed disposable seeding system comprising a vessel with fluid comprising blood or enriched cells, b) inserting a graft or scaffold over the mandrel of the seeding chamber, c) filling the seeding chamber with the fluid, d) applying negative pressure to a lower mandrel outlet and emptying the chamber to about half way, e) inverting the chamber, and f) applying negative pressure and emptying the chamber through an upper outlet port.

The fluid may be blood, bone marrow aspirate, or cell extract from the blood or bone marrow. The fluid may be of a volume between about 10 ml and 200 ml, such as between about 25 ml, about 50 ml, about 75 ml, about 100 ml, about 125 ml, about 150 ml, about 175 ml, and about 200 ml. The fluid typically has a white blood cell (WBC) concentration has between about $10^5$ cells/ml and $10^8$ cells/ml, preferably between about $10^6$ and $10^8$ cells/ml, more preferably between about $10^6$ and $10^7$ cells/ml.

After seeding, the graft or scaffold typically has a cell density between about 0.1 x$10^3$ cells/mm$^2$ and $10^5$ cells/mm$^2$, inclusive, along the length of the scaffold, preferably between about 0.1 x$10^3$ cells/mm$^2$ and $10^4$ cells/mm$^2$, inclusive, along the length of the scaffold, most preferably between 1 x$10^3$ cells/mm$^2$ and $10^4$ cells/mm$^2$ , inclusive, along the length of the scaffold.

Typically the graft is contacted with fluid for a period of a period of time between about 1 min and 15 min, preferably between about 1 min and 10 min, most preferably between about 1 min and 7 min. In most preferred embodiments, seeding is complete is about 15 min, about 7 min, or about 1 min.

## C. Grafts or Scaffolds with Reversible Stenosis

Typically, the graft or the scaffold is positioned between the mandrel and the housing. The graft or the scaffold is generally polymeric and porous, made of biodegradable polymer. The graft or the scaffold may have an inner surface structure and an outer surface structure for inducing spontaneous reversal of stenosis. The graft or the scaffold may have an average pore size on its inner surface and that is different from the average pore size on its outer surface. For example, the average pore size on the inner surface may be between about 35 μm and 50 μm, preferably between about 38 μm and 50 μm, most preferably between about 38 μm and 45 μm. The average pore size on the outer surface may be between about 25 μm and 45 μm, preferably between about 27 μm and 43 μm, most preferably between about 30 μm and 43 μm. The surface porosity may be between about 0.6 and 0.95, preferably between about 0.7 and 0.9, most preferably between about 0.8 and 0.9, of the surface area of the inner surface and/or the outer surface.

Typically, the graft or the scaffold is formed from knitted polymeric fibers. In some aspects, the fibers may be organized in fiber bundles. The fiber bundles may be knitted, such as knitted in a weft pattern. The fiber diameter may be between about 5 nm and 30 nm. Typically, the knitted pattern forms polymer fiber layers arranged axially and polymer fiber peaks arranged circumferentially. The separation between the layers may be between about 0.5 mm and 2 mm, preferably between about 0.5 mm and 1.5 mm, most preferably about 1 mm. The separation between the peaks may be between about 0.5 mm and 2 mm, preferably between about 0.5 mm and 1.5 mm, most preferably about 1 mm.

The grafts or the scaffolds may include a fibrous polymer coating. Typically, the TEVG or the scaffolds have an inner diameter between about 14 mm and 22 mm, thickness between 0.1 mm and 3 mm, and a length between about 5 cm and 15 cm. The TEVG or the scaffold are typically biodegradable and are substantially degraded within about six months following implantation. For example, by six months following implantation, the graft has been reduced by greater than about 80% by weight, greater than about 80% by surface area, or greater than about 80% by thickness, of the scaffold.

The polymeric vascular grafts or scaffolds typically include an effective amount of viable cells to reduce or prevent post-operative stenosis of the graft relative to the graft without the cells or with less cells. Typically, the graft has attached thereto an amount of viable cells at a cell density between about $0.1 \times 10^3$ cells/$mm^2$ and $10 \times 10^4$ cells/$mm^2$, inclusive, along the length of the scaffold, preferably between about $0.1 \times 10^3$ cells/$mm^2$ and $10 \times 10^3$ cells/$mm^2$, inclusive, along the length of the scaffold, most preferably between $1 \times 10^3$ cells/$mm^2$ and $10 \times 10^3$ cells/$mm^2$ , inclusive, along the length of the scaffold.

Preferred cells are obtained from the patient's bone marrow. In preferred embodiments, the vascular graft is seeded with viable autologous cells. In a particular embodiment, the cells are human bone marrow mononuclear cells. The polymeric vascular graft or scaffold can include one or more additional agents selected from the group consisting of anti-neointima agents, chemotherapeutic agents, steroidal and non-steroidal anti-inflammatories, conventional immunotherapeutic agents, immune-suppressants, cytokines, chemokines, and growth factors.

## 1. Polymers

TEGV scaffolds can be formed of one or more polymers. In some embodiments, the polymers are biodegradable. In other embodiments, the polymers are non-biodegradable. In some embodiments TEVG are formed from a mixture of more than a single polymer. When biodegradable polymers are used, mixtures of biodegradable and non-biodegradable polymers can be used, for example, to provide long-lasting TEVG implants, as desired.

In certain embodiments, TEVGs are a three-dimensional matrix formed of polymeric (homopolymer and/or copolymer) fibers that are assembled in a woven or non-woven mesh, in random or aligned configurations. Preferably, the nanofiber materials are FDA approved biodegradable nanofiber materials.

The fibers in the scaffold matrix can be of any desired size, but generally are between about 1.5 mm and 1 nm. In certain embodiments, the fibers are nanoscale (*i.e.,* from about 1 nm to about 1000 nm) and/or microscale (from about 1 $\mu$m to about 1000 $\mu$m).

Polymers useful for creating a scaffold for use in formation of TEVGs may be inorganic (*e.g.,* siloxane, sulfur chains, black phosphorus, boron-nitrogen, silicones) or organic (meaning containing carbon). Organic polymers may be natural (*e.g.,* polysaccharides, such as starch, cellulose, pectin, seaweed gums, vegetable gums; polypeptides, such as casein, albumin, globulin, keratin, collagen, nucleic acid, and hydrocarbons), synthetic (such as thermoplastics, unvulcanized elastomers, nylon, polyvinyl chloride, linear polyethylene, polystyrene, polypropylene, polyurethane, acrylate resins); thermosetting (*e.g.*, vulcanized elastomers, crosslinked polyethylene, phenolics, alkyds, polyesters), and semisynthetic (*e.g.*, cellulosics, such as rayon, methylcellulose, cellulose acetate; and modified starches)). In addition, useful scaffolds may include hydrogels formed from water soluble or water insoluble cellulose compounds. As would be readily understood by the skilled artisan, the particular type and composition of scaffold will vary depending upon the desired application. However, it is generally preferred that the polymeric material in the scaffold be biocompatible (*i.e.*, will not elicit an unwanted immune reaction). In certain embodiments, the scaffold is biodegradable. Exemplary degradable polymers include poly(lactic acid-glycolic acid), poly(lactic acid), poly(glycolic acid), poly(orthoesters), poly(phosphazenes), polycaprolactones, or polyamides. In a preferred embodiment, the polymer is poly(lactic acid-glycolic acid). In one embodiment, the TEVGs are formed from a biodegradable tubular scaffold fabricated from a polyglycolic acid-fiber tube. The tube can be coated with a copolymer such as a 50:50 poly lactic acid (PLA) and poly-caprolactone copolymer.

In one embodiment, the grafts are formed from a felt or sheet like material of the polymer that can be formed into a tubular scaffold. For example, the device could be fabricated as a nonwoven, woven or knitted structure from extruded polymeric fibers. Typically, the polymeric sheet is formed using any textile construction, including, but not limited to, weaves, knits, braids or filament windings. Any suitable method, such as electrospinning, can be used to fabricate the nonwoven or woven polymeric textile.

The polymers and fabrication methods selected to fabricate the polymeric vascular grafts are suitable to produce grafts with biomechanical properties suitable for use as vascular scaffolds. Biomechanical properties that are important for vascular graft function include initial burst pressure, suture retention strength and elasticity. In one embodiment, the initial burst pressure of the polymeric vascular graft is between about 1,500 mmHg and about 50,000 mmHg, preferably between about 2,000 mmHg and about 10,000 mmHg. In another embodiment, the polymeric vascular grafts possess suture retention strengths between about 1 N and about 5 N, preferably between about 2 N and about 4 N. In another embodiment, the intrinsic elasticity of the vascular grafts is between about 10 MPa and about 50 MPa, preferably between about 15 MPa and about 40 MPa. In another embodiment, the initial tensile strength of the vascular grafts is between about 1 MPa and about 10 MPa, preferably between about 3 MPa and about 6 MPa.

## 2. Cells

In certain embodiments, the TEVGs scaffolds include one or more types of cells. In some embodiments, one or more types of cells are included within the lumen of the TEVG, within porous spaces throughout the walls of the TEVG, on the exterior and surface of the TEVG, or combinations. Typically, cells are attached to the surface of the TEVG, either directly, or through one or more accessory substances. In some embodiments, the cells are autologous cells, derived from one or more tissues of the intended recipient of the cell-seeded TEVG. In other embodiments, the cells are exogenous to the intended recipient of the graft. The cells can be undifferentiated cells, such as pluripotent stem cells, or differentiated cells. In preferred embodiments, the cells are viable human cells. Exemplary cell types for inclusion in TEVGs include white blood cells (WBC), such as monocytes lymphocytes, neutrophils, basophils, eosinophils; fibroblasts; myofibroblasts fibroblast cells; smooth muscle cells; bone marrow progenitor cells; red blood cells; embryonic stem cells; and combinations. White blood cells are made in bone marrow. In a particular embodiment, autologous bone marrow mononuclear cells (BM-MNCs) are included within the TEVG.

Cells for use with the TEVGs can be obtained from multiple sources. Methods for isolating and optionally manipulating one or more cell types from mixtures of cells are known in the art. In an exemplary embodiment, bone marrow is collected from the one or more long bones (*e.g.*, femurs and/or tibias) of a subject (e.g., the intended recipient of the TEVG) and mononuclear cells are isolated using the density centrifugation method (Lee, et al. J Vis Exp. (88), (2014); Udelsman, et al. Tissue Eng Part C Methods. 17(7), 731-736 (2011)).

### a. Seeding Dose

Typically, the seeding chambers provide the maximum uniform seeding density along the length of the graft or scaffold with the minimum volume of cell-containing fluid (blood, bone marrow aspirate, or mononuclear cell (MNC)-enriched fraction).

Typically, the amount of cells seeded into the TEVG is directly proportional to post-operative graft patency. Therefore, in preferred embodiments, TEVGs are seeded with a sufficient amount of cells effective to enhance the patency, or reduce the rate of post-operative stenosis of the TEVG. Methods for manually seeding TEVGs with cells are known in the art (Udelsman, et al. Tissue Eng Part C Methods. 17(7), 731-736 (2011)).

The optimal number of cells seeded can vary according to the cell type, and the size and shape of the TEVG, as well as the intended use. TEVGs are typically contacted with an amount of cells between $1.0 \times 10^6$ cells and $500 \times 10^6$ cells, inclusive, preferably between $3.0 \times 10^6$ cells and $250 \times 10^6$ cells.

When seeded, the resulting seeding density is uniform along the length of the scaffold. The seeding density may be between about $0.1 \times 10^3$ cells/mm$^2$ and $10 \times 10^4$ cells/mm$^2$ along the length of the scaffold. In preferred embodiments, TEVG are seeded at a density of between about $0.1 \times 10^3$ cells/mm$^2$ and $10 \times 10^3$ cells/mm$^2$ , inclusive, along the length of the scaffold preferably between $1 \times 10^3$ cells/mm$^2$ and $10 \times 10^3$ cells/mm$^2$ , inclusive, along the length of the scaffold.

In some embodiments, TEVGs are seeded with cells in solution having a concentration of between about $0.1 \times 10^4$ cells/ml and $10 \times 10^7$ cells/ml, inclusive. Typical volumes of cell solutions range between 1 ml and 100 ml, inclusive, preferably between 10 ml and 100 ml, such as 10 ml, 50 ml, 75 ml, or 100 ml.

Preferably, TEVGs are seeded with cells in an amount sufficient to yield a cell density of between $0.1 \times 10^3$ cells/mm$^2$ and $10.0 \times 10^4$ cells/mm$^2$, preferably between $1.0 \times 10^3$ cells/mm$^2$ and $10.0 \times 10^3$ cells/mm$^2$. Therefore, seeding chambers allow the grafts or scaffolds to be seeded using the minimum amount of bone marrow harvested from the patient and achieve uniform cell density along the length of the graft or scaffold.

When autologous bone marrow mononuclear cells (BM-MNCs) cells are prepared for seeding, the cells from 5 ml/kg of bone marrow are typically provide a sufficient seeding dose. From a clinical perspective, up to 20 ml/kg of bone marrow can be harvested from an individual without incurring significant adverse effects and is routinely used for harvesting bone marrow for bone marrow transplantations.

### 3. Additional Active Agents

It has been established that TEVG seeded with bone marrow-derived mononuclear cells reduce and prevent the incidence of post-operative stenosis *via* a paracrine effect. Advantages of cell seeding include the release signals in response to the body's feedback mechanism, unlike the drug-eluting scaffolds which release the drug regardless of feedback. Therefore, in some embodiments, cell-seeded TEVG are used in combination with one or more non-cell based synthetic or non-synthetic compounds that replicate the paracrine effect of seeded cells.

The TEVGs can include additional active agents, for example, that enhance the adhesion of cells to the vascular graft, or reduce the incidence of post-operative stenosis of the graft following insertion. Active gents include, but are not limited to, anti-neointima agents, chemotherapeutic agents, steroidal and non-steroidal anti-inflammatories, conventional immunotherapeutic agents, immune-suppressants, cytokines, chemokines, and growth factors.

Use of growth factors to stimulate bone marrow growth represents an additional strategy for increasing the yield of BM-MNC. Therefore, in some embodiments, the TEVGS include growth factors. Exemplary growth factors for incorporating into TEVGS include growth factors released in the physiological response to tissue injury, which stimulate the deposition of extracellular matrix, such as Platelet-Derived Growth Factor (PDGF), a potent chemotactic agent, and Transforming Growth Factor beta (TGF-$\beta$).

## III. Methods of Making

### A. Seeding Chambers

The one or more of the component parts of the seeding system are custom designed such that the assembly is optimally sized to accommodate the dimensions of the TEVG. Preferably, the system, or parts of the system are fabricated using 3D printing of suitable materials. In an exemplary embodiment, one or more of the component parts of the seeding chamber of the system illustrated in Figures 2A-4C is fabricated by 3D printing of suitable materials. In a particular embodiment, the components that are 3D-printed include one or more of a seeding chamber (e.g., seeding chambers **700** and **800**), a suction rod, and a clip, a cap, and a mandrel. The components can be assembled into a seeding chamber assembly with a manufactured scaffold. The dimensions of the component parts of the seeding system can be varied according to those desired, for example, by the dimensions of the vascular graft.

Suitable materials for forming the different components of the seeding systems include polymers and metals, including, but not limited to, stainless steel, iridium, platinum, gold, tungsten, tantalum, palladium, silver, niobium, zirconium, aluminum, copper, indium, ruthenium, molybdenum, niobium, tin, cobalt, nickel, zinc, iron, gallium, manganese, chromium, titanium, aluminum, vanadium, and carbon, as well as combinations, alloys, and/or laminations thereof.

### B. Mandrel

Mandrels for use in the formation of TEVGs can be fabricated using means known in the art. Exemplary methods for the fabrication of mandrels include 3D printing. In some embodiments, the final mandrel design is converted to a suitable computer-readable format for 3D fabrication. An exemplary computer-readable format is STL format. Suitable materials for 3D printing of mandrel models include polymers and metals and carbon, as well as combinations, alloys, and/or laminations thereof. In some embodiments, the mandrel is made of a liquefiable material, thereby allowing the release of the mandrel from the graft in an easy fashion. The use of liquefiable mandrels also allows for forming complex shapes of the graft.

### C. Methods of Making TEVGs

Methods of fabricating TEVGs based on a template structure, such as a custom-designed mandrel, are described herein. TEVGs can be fabricated using any appropriate method, such as electrospinning, stamping, templating, molding, weaving and combinations melt processing, solvent processing, leaching, foaming, extrusion, injection molding, compression molding, blow molding, spray drying, extrusion coating, and spinning of fibers with subsequent processing into woven, non-woven, or knitted constructs.

In some embodiments, TEVGs are fabricated to include pores in the graft. Pores can be derived by any suitable method, including salt leaching, sublimation, solvent evaporation, spray drying, foaming, processing of the materials into fibers and subsequent processing into woven or non-woven devices. In a preferred embodiment, the fiber matrix of the scaffold includes pores of a suitable size to allow cells to adhere and grow and/or differentiate. Since the diameter of a cell is approximately 10 $\mu$m to 20 $\mu$m, pore sizes within this range are desired in certain embodiments. Preferably, the pores of the device are between 5 and 500 $\mu$m, more preferably between 5 and 250 $\mu$m, more preferably between 5 and 100 $\mu$m, in diameter. In certain embodiments, the polymeric scaffolds are generated or fabricated in order to more closely mimic the structure and composition of the natural extracellular matrix in order to promote growth and differentiation of the seeded cell and to facilitate transplantation and/or implantation of the scaffold or cells grown on the same.

In a preferred embodiment, TEVGs are fabricated by electrospinning of a stock solution containing one or more polymers. Typically, one or more polymers used to fabricate TEVGs are biodegradable polymers.

### D. Methods of Seeding TEVGs

Typically, the seeding chamber, such as the chamber 700 or 800, is connected to a closed disposable seeding system containing a fluid with cells.

As claimed herein, in a fourth aspect of the present invention, there is provided a method for seeding a graft or scaffold with cells, the method comprising: a) connecting the seeding chamber having a "flip" design in accordance with the first aspect of present invention, to a closed disposable seeding system comprising a vessel with fluid comprising blood or enriched cells, b) inserting a graft or scaffold over the mandrel of the seeding chamber, c) filling the seeding chamber with the fluid, d) applying negative pressure to a lower mandrel outlet and emptying the chamber to about half way,e) inverting the chamber, and f) applying negative pressure and emptying the chamber through an upper outlet port.

Typically, the number of cells used to contact the graft or scaffold may be proportional to the surface area of the graft, wherein the number of cells is between about $1.0 \times 10^4$ cells/mm$^2$ graft and $1.0 \times 10^6$ cells/mm$^2$ graft, inclusive, preferably between $0.7 \times 10^5$ cells/mm$^2$ graft and $7.0 \times 10^5$ cells/mm$^2$ graft, inclusive. In some embodiments, the polymeric vascular grafts or scaffolds are contacted with an amount of cells between $0.5 \times 10^6$ cells and $500 \times 10^6$ cells, inclusive, preferably between $1.0 \times 10^6$ cells and $100 \times 10^6$ cells. In preferred embodiments, the graft is contacted with the cells for less than 3 hours, preferably less than 2 hours, such as about 30 min, about 20 min, about 15 min, about 7 min, about 5 min, or about 1 min. The contacting is typically carried out within a sterile, closed seeding chamber.

Accordingly, the patency of a polymeric vascular graft or scaffold can be increased, by administering an effective amount of viable cells onto the graft or scaffold to reduce the infiltration of macrophages to the graft, to promote the recruitment of host cells to the graft or to reduce or prevent platelet activation.

The seeded graft or scaffold produced by the methods of the fourth aspect of the present invention are useful for reducing or preventing post-operative stenosis in a subject. The subject can be a subject at risk of or has restenosis or other vascular proliferation disorder. For example, in some embodiments, the subject has undergone, is undergoing, or will undergo vascular trauma, angioplasty, vascular surgery, or transplantation arteriopathy. The seeded graft or scaffold can be used to reduce neointima formation, stenosis or restenosis, reduce or prevent thrombosis, or any combination thereof in a subject relative to an untreated control subject.

Restenosis means the recurrence of a treated coronary artery stenosis over time. Restenosis is most commonly defined as luminal renarrowing of greater than 50% (binary angiographic restenosis), either within the stent (in-stent restenosis) or within the stent and including 5 mm proximal or distal to the stent margin (in-segment restenosis) on follow-up angiography (typically 6 or 9 months later). Restenosis may manifests itself clinically over the 1- to 6-month period following a PCI (Percutaneous Coronary Intervention).

Customizable systems and compositions for seeding of cells into a vascular graft or scaffold are described in US Patent No. 9,090,863 and US Application Publication No. US 2018/0353649.

### IV. Methods of Using

Typically, the seeding chambers are used for fast and efficient seeding of the grafts and scaffolds. The seeded scaffolds are then used in cardiovascular surgeries to repair or replace damaged vessels.

### A. Methods of Using the Seeding Chambers

#### 1. Flip Seeding Chamber

Methods of using the flip seeding chamber of the first aspect of the present invention include the following steps. The chamber is filled, a negative pressure is applied, the chamber has 50% of the volume drained (at which point the bottom ~50% of the scaffold is saturated with mononuclear cells (MNCs)), the negative pressure is interrupted, the device is inverted, and the negative pressure is re-introduced, allowing the remaining 50% of the volume to be drawn through the "upper" half of the scaffold, which then also becomes saturated with MNCs. Therefore, the bottom and top halves of the scaffold saturate sequentially, resulting in a completely saturated scaffold with minimal MNC loss (high seeding efficiency).

The negative pressure may be provided by a syringe, pump, or vacuum source.

#### 2. Capacitor Seeding Chamber

Methods of using the alternative capacitor seeding chamber design, which is described but not claimed herein, include the following steps. The mandrel and scaffold assembly are carefully placed into the seeding chamber. The seeding chamber is very narrow as compared to the assembled mandrel/scaffold assembly. It is designed to hold a minimum volume of MNC solution. The cell seeding chamber may have a gap between about 1 mm and about 10 mm, more preferably between about 1 mm and 5 mm between the between the suction rod or the mandrel and the housing.

After the mandrel and chamber are secured together, the MNCs are introduced into the device. The fluid quickly fills the chamber and "overflows" into an IV bag located above the chamber. After the entire volume of MNCs have been introduced into both the seeding chamber and overflow (capacitor) IV bag, a negative pressure is applied. The MNC fluid begins to pass through the scaffold, and the entire scaffold remains submerged in MNCs as the fluid level is lowered in the IV bag. It is not until the very end of the seeding process that the fluid is completely drained from the IV bag and the fluid level eventually falls below the top of the seeding device. There is only a very small amount of time in which the fluid is drained from the top of the scaffold exposing the scaffold to filtered air while the bottom of the scaffold remains submerged. Thus, the top and bottom of the scaffold remained submerged throughout the majority of the seeding process. Only for a small fraction of the MNC solution is the top exposed while the bottom is submerged. Therefore, the seeding gradient is minimized along the length of the scaffold.

The negative pressure may be provided by a syringe, pump, or vacuum source.

**B. Methods of Using Grafts or Scaffolds**

It has been established that the cell-seeding dose on tissue engineered vascular graft (TEVG) is an effect-dependent variable for improving the performance and utility of the graft, regardless of cell incubation time. Typically, TEVGs are seeded with cells prior to implanting into a subject. Typically, the cells are autologous cells from the intended recipient, and the methods of seeding can include the step of harvesting the cells from the recipient. One or more cell types can be isolated from a mixture of cells using any techniques known in the art. Therefore, the methods can also include the step of isolating or purifying the cells prior to application (i.e., seeding).

Seeding of TEVG with cells is carried out using a kit or device, such as the closed disposable seeding system. The closed disposable seeding system may include any one of flip seeding chamber or capacitor seeding chamber for seeding the cells onto the graft or scaffold. Preferably, the kit or device enables sterile and efficient seeding of TEVG with a controllable amount of cells.

**1. Methods of Use of TEVGs Seeded with Cells**

TEVGs seeded with cells can reduce or prevent the rate of post-operative stenosis of the TEVG, relative to the rate of stenosis in the equivalent TEVG in the absence of cells. Therefore, TEVGs can be seeded with an effective amount of cells to reduce or prevent one or more of the immune processes associated with development of post-operative stenosis, including inflammation.

Tissue repair has four distinct stages, including: a) clotting/coagulation; b) inflammation; c) fibroblast migration/proliferation; and d) a final remodeling phase where normal tissue architecture is restored. In the earliest stages after tissue damage, epithelial cells and/or endothelial cells release inflammatory mediators that initiate an antifibrinolytic-coagulation cascade that triggers clotting and development of a provisional extracellular matrix (ECM). Aggregation and subsequent degranulation of platelets promotes blood vessel dilation and increased permeability, allowing efficient recruitment of inflammatory cells such as neutrophils, macrophages, lymphocytes, and eosinophils to the damaged tissue. Neutrophils are the most abundant inflammatory cell at the earliest stages of wound healing, but are quickly replaced by macrophages after neutrophil degranulation. Activated macrophages and neutrophils debride the wound, eliminate any invading organisms and produce a variety of cytokines and chemokines that amplify the inflammatory response as well as trigger fibroblast proliferation and recruitment. Upon activation, fibroblasts transform into myofibroblasts that secrete $\alpha$-smooth muscle actin and ECM components. Finally, in the remodeling phase epithelial/endothelial cells divide and migrate over the temporary matrix to regenerate the damaged tissue. Thus, healing and neotissue generation is a finely regulated process that balances the need to regenerate tissue and thicken blood vessel walls, without excessive thickening and stenosis or fibrosis.

**a. Macrophages**

It has been shown that the presence of circulating monocytes and infiltrating macrophages is critical for wound healing and neotissue development (Arras, et al., J Clin Invest, 101(1): 40-50 (1998)). However, the extent of macrophage infiltration at a site of tissue damage has also been correlated with proliferative dysregulation and neointima formation (Hibino, et al., FASEB J. 25(12):4253-63 (2011)). Further, numerous studies have indicated that macrophages and fibroblasts are the main effector cells involved in the pathogenesis of fibrosis (reviewed in Wynn, Nat Rev Immunol. 4(8):583-94 (2004)).

Following vascular damage, inflammatory monocyte cells (CD16-hi, CD64-hi and CD14-hi in humans; CD115+, CD11b+ and Ly6c-hi in mice) are recruited to the damaged tissue and differentiate into activated macrophages (Emr1-hi in humans; F4/80-hi in mice) upon exposure to local growth factors, pro-inflammatory cytokines and microbial compounds (Geissmann et al., Science 327: 656-661 (2010)). Excessive macrophage infiltration results in stenosis, whilst complete inhibition of macrophage infiltration prevents neotissue formation (Hibino, et al., FASEB J. 25(12):4253-63 (2011).

Two distinct states of polarized activation for macrophages have been defined: the classically activated (M1) macrophage phenotype and the alternatively activated (M2) macrophage phenotype (Gordon and Taylor, Nat. Rev. Immunol. 5: 953-964 (2005); Mantovani et al., Trends Immunol. 23: 549-555 (2002)). The role of the classically activated (M1) macrophage is an effector cell in TH1 cellular immune responses, whereas the alternatively activated (M2) macrophage appears to be involved in immunosuppression and wound healing/tissue repair. M1 and M2 macrophages have distinct chemokine and chemokine receptor profiles, with M1 secreting the TH1 cell-attracting chemokines CXCL9 and CXCL10, and with M2 macrophages expressing chemokines CCL17, CCL22 and CCL24.

The presence of M2 macrophages has been associated with neointima development and stenosis (Hibino, et al., FASEB J. 25(12):4253-63 (2011)). The correlation between the extent of macrophage infiltration, neotissue formation and stenosis at certain time points following tissue graft implantation provides means to prevent stenosis through modulation of macrophage activity.

Further, macrophages are typically located close to collagen-producing myofibroblast cells, and it has been shown that monocyte-derived macrophages critically perpetuate inflammatory responses after injury as a prerequisite for fibrosis (Wynn and Barron, Semin Liver Dis., 30(3):245-257 (2010)). Macrophages produce pro-fibrotic mediators that activate fibroblasts, including platelet-derived growth factor (PDGF), a potent chemotactic agent, and transforming growth factor beta (TGF-B). Specifically, a marked increase of the non-classical M2 (CD14+, CD16+) subset of macrophages has been correlated with pro-inflammatory cytokines and clinical progression in patients suffering from chronic liver disease. During fibrosis progression, monocyte-derived macrophages release cytokines perpetuating chronic inflammation as well as directly activate hepatic stellate cells (HSCs), resulting in their proliferation and trans-differentiation into collagen-producing myofibroblasts (Zimmermann, et al., PLOS One, 5(6):e11049 (2010)).

### b. Platelets

Aggregated platelets assist the repair of blood vessels by secreting chemicals that attract fibroblasts from surrounding connective tissue into the wounded area to heal the wound or, in the case of dysregulated inflammatory responses, form scar tissue. In response to tissue injury, platelets become activated and release a multitude of growth factors which stimulate the deposition of extracellular matrix, such as platelet-derived growth factor (PDGF), a potent chemotactic agent, as well as transforming growth factor beta (TGF-$\beta$). Both of these growth factors have been shown to play a significant role in the repair and regeneration of connective tissues. PDGF functions as a primary mitogen and chemo-attractant which significantly augments the influx of fibroblasts and inflammatory cells, as well as stimulating cell proliferation and gene expression. PDGF enables leukocytes to firmly attach to the vessel wall and finally to transmigrate into the subendothelial tissue. However, the platelet-derived chemokines are also known to induce smooth muscle cell (SMC) proliferation and play a role in neointimal proliferation and organ fibrosis (Chandrasekar, et al., J Am College Cardiology, Vol 35, No. 3, pp. 555-562 (2000)). Increased expression of PDGF and its receptors is associated with scleroderma lung and skin tissue. Specifically, there is evidence for an autocrine PDGF-receptor mediated signaling loop in scleroderma lung and skin fibroblasts, implicating both TGF-$\beta$ and PDGF pathways in chronic fibrosis in scleroderma (Trojanowska, Rheumatology;47:v2-v4 (2008)). In addition, deregulation of PDGF signaling is associated with cardiovascular indications such as pulmonary hypertension, and atherosclerosis.

Media layer smooth muscle cell (SMC) proliferation and migration in response to injury-induced PDGF are essential events contributing to neointimal thickening (Fingerle, et al., Proc Natl Acad Sci., 86:8412 (1989); Clowes, et al., Circ. Res., 56:139-145 (1985)) which eventually leads to blood vessel narrowing and stenosis.

Other healing-associated growth factors released by platelets include basic fibroblast growth factor, insulin-like growth factor 1, platelet-derived epidermal growth factor, and vascular endothelial growth factor.

The TEVGs can be seeded with an effective amount of cells to create a pro-regenerative immune environment that enhances wound healing and prevents stenosis. The TEVG can also be seeded with an effective amount of cells to modulate platelet activity and function. Thus, the TEVG can also be seeded with an effective amount of cells to reduce or prevent the biological functions of platelets, such as platelet aggregation and the production/expression of platelet derived growth factor (PDGF).

Methods of using the tissue engineering vascular grafts seeded with cells to reduce post-operative stenosis of the graft include surgically implanting, or otherwise administering, the cell-seeded grafts to within a patient. Typically, the method of implanting includes attaching the graft to a section of an artery that is to be replaced or augmented. Methods of attaching vascular grafts are known in the art. The methods typically reduce or inhibit the infiltration of macrophage cells, or the conversion of macrophage cells from M1 to M2 phenotype, or both, compared to a control, such as an equivalent graft that is not seeded with cells, or seeded with fewer cells. In some embodiments, the methods reduce or inhibit proliferation of macrophage cells without reducing or inhibiting vascular neotissue development. A subject can have stenosis, restenosis or other vascular proliferation disorders, or be identified as being at risk for restenosis or other vascular proliferation disorders, for example subjects who have undergone, are undergoing, or will undergo a vascular trauma, angioplasty, surgery, or transplantation arteriopathy, *etc.* Any of the methods described can include the step of identifying a subject in need of treatment.

The present invention will be further understood by reference to the following non-limiting examples.

**Examples**

**Example 1. Seeding chambers for uniform cell seeding of grafts.**

<u>Materials and Methods</u>

U.S. Patent No. 9,090,863 describes how a closed, disposable tissue engineered vascular graft seeding device was used to seed mononuclear cells (MNCs) onto an implantable scaffold.

Every scaffold seeded using this method had a gradient (distribution) of MNCs along the longitudinal axis of the graft (meaning there were less cells seeded on the top of the graft than the bottom of the graft). With new concentrations and seeding volumes, seeded scaffolds reached a point of saturation towards the bottom 3 cm of the graft (Figures 2A and 2B), which did not permit further cell seeding resulting in waste of bone marrow and potential risk to patients.

The entire graft (13 cm in length) save 5 mm from the top and the bottom graft was seeded with enough MNCs to meet release criteria, however, there were many more MNCs found in the lower sections of the graft than the upper sections. This example presents two seeding chambers - flip and capacitor, for seeding devices to eliminate this disparity.

**The Flip seeding chamber**

The 'Flip' seeding chamber eliminates the variability of the distribution of MNCs on the scaffold. The central premise behind the device is to vary the cross section of the seeding chamber along its length.

Figures 3A-3E show an example of a flip seeding chamber. The shape of the chamber resembles that of the inverse of an hour glass, with a bulge (greatest cross section) approximately 40% of the way down the height of the device. Both the top and the bottom of the device narrow to accommodate the mandrel with the scaffold mounted to it, along with a very minimal volume for part clearance and excess liquid.

The effect of the change in cross section is that it effectively slows the speed at which the MNCs are drawn through the least seeded portions of the scaffold.

The chamber is filled, a vacuum applied, the chamber has 50% of the volume drained (at which point the bottom ~50% of the scaffold is saturated with MNCs), the vacuum is interrupted, the device is inverted, and the vacuum is re-introduced, allowing the remaining 50% of the volume to be drawn through the "upper" half of the scaffold, which then also becomes saturated with MNCs. Via this method, the bottom and top halves of the scaffold saturate sequentially, resulting in a completely saturated scaffold with minimal MNC loss (high seeding efficiency).

In addition, the mandrel for this design has two vacuum nubs - one to draw negative pressure in the upright orientation, and one to draw negative pressure in the inverted orientation.

***Method of seeding a graft with flip chamber***

1. The scaffold is secured on the mandrel (upper portion of the device).
2. The mandrel (upper) is secured to the chamber (lower) part of the device by rotating it counter clockwise until it compresses a sealing o-ring
3. All necessary fixtures, tubes, and air vents are placed on the device
4. The chamber is filled with MNCs
5. Negative pressure is introduced to the lower mandrel outlet.
6. The chamber has 50% of the volume pulled through it.
7. The vacuum is interrupted.
8. The device is inverted
9. The vacuum is re-introduced
10. The remaining 50% of volume is drawn through the "upper" portion of the scaffold.
11. The device is disassembled and the scaffold is now ready for implantation.

Total seeding time was 7 min (7:01±0.03 min).

After seeding, the seeded scaffold was cut into 1 cm -wide rings along the length of the scaffold forming 12 rings. The number of cells in each ring was counted and the data are shown in Figure 5. The cells were counted using PicoGreen dsDNA assay, and the counts are presented as cells/mm$^3$.

**The Capacitor seeding chamber**

The 'Capacitor' seeding chamber eliminates the variability of the distribution of MNCs on the scaffold. The central premise behind the device is to reduce the MNC gradient by exposing the entire scaffold to the MNC seeding process for as long as possible.

Figures 4A-4C show an example of a capacitor seeding chamber. In this chamber, the mandrel has a scaffold mounted to it. Then, the scaffold and mandrel assembly are carefully placed into the seeding chamber. The seeding chamber is very narrow as compared to the assembled mandrel/scaffold assembly. It is designed to hold a minimum volume of MNC solution. The minimum and maximum gap between the scaffold and the wall of the chamber for uniform seeding may be 1 mm and 10 mm, such as 1 mm and 5 mm.

After the mandrel and chamber are secured together, the MNCs are introduced into the device. The liquid quickly fills the chamber and "overflows" into an IV bag located above the chamber.

After the entire volume of MNCs have been introduced into both the seeding chamber and overflow (capacitor) IV bag, the vacuum is applied. The MNC liquid begins to pass through the scaffold, and the entire scaffold remains submerged in MNCs as the liquid level is lowered in the IV bag. It is not until the very end of the seeding process that the liquid is completely drained from the IV bag and the fluid level eventually falls below the top of the seeding device. There is only a very small amount of time in which the liquid is drained from the top of the scaffold exposing the scaffold to filtered air while the bottom of the scaffold remains submerged. Thus, the top and bottom of the scaffold remained submerged throughout the majority of the seeding process. Only for a small fraction of the MNC solution is the top exposed while the bottom is submerged. Therefore, the seeding gradient is minimized along the length of the scaffold.

***Method of seeding a graft with capacitor chamber***

    1. filling the IV bag
    2. filling the chamber
    3. Bleed air from chamber
    4. Fill chamber with blood, covering scaffold
    5. introduce vacuum, vacuum drawing fluid through scaffold
    6. drain chamber
    7. remove seeded scaffold

Total seeding time was 1 min 15 seconds.

After seeding, the seeded scaffold was cut into 1 cm -wide rings along the length of the scaffold forming 12 rings. The number of cells in each ring was counted and the data are shown in Figure 5.

**Results**

The results show uniform seeding of the scaffolds seeded with either flip seeding chamber (1) or capacitor seeding chamber (2) in Figure 5.

**Example 2. Scaffold parameters providing spontaneous reversal of stenosis.**

A computational model was developed to simulate neovessel formation. This computational model was initially formulated from data collected in prior studies of TEVG development in mouse models, and it successfully described and predicted neovessel formation over a 2-year period. Presented is the first analysis of data from a United States clinical trial as well as computational simulations that suggested that the early stenosis observed in a prior clinical trial may have reversed naturally without intervention. Presented are also experiments using an established large animal model to test the simulation-generated suggestion that a transient period of TEVG narrowing would develop and subsequently resolve spontaneously as part of the natural history of neovessel formation. Characterized are the evolving geometry, composition, and biomechanical properties of TEVGs up to 1.5 years post-implantation in a large animal model. The data validated the primary predictions of the computational model. Comparisons of *in vivo* observations to results from the computational model further enabled to refine values of the model parameters and thereby to glean increased insight into the mechanisms that underlie the transformation of TEVGs from scaffolds seeded with autologous cells into living neovessels capable of growth and remodeling.

**Materials and Methods**

*TEVG*

*Scaffold characterization*

Scaffolds were characterized using Scanning Electron Microscopy (SEM). Samples of nonimplanted scaffold were cut along the axial direction to create 0.5 cm squares that were mounted on SEM stages with carbon tape. Samples were sputter coated with gold to 3-nm thickness under vacuum in argon gas and imaged on a Hitachi S4800 SEM at 5kV and 10mA. Images were analyzed with FIJI image analysis software. Pore size was calculated from 7 SEM images at 100x. Fiber diameter was calculated on average by at least 5 PGA fibers.

*Release Criteria and Post Process Testing*

Samples were obtained from cells (0.2mL aliquots) and seeded scaffold (5x5mm sections), and subject to release and post-process monitoring. Cell count and viability were performed using trypan blue exclusion and a hemocytometer. FACS was performed using FITC-CD45 and 7AAD to determine the number of leukocytes and cell viability. Seeding efficacy was determined by quantifying the number of cells in samples obtained from the pre-seeding and post-seeding solutions using a hemocytometer and then calculating the difference in the number of cells in the pre-seeding and post-seeding solutions divided by the number of cells in the pre-seeding solution.

**Clinical Trial**

*Study design*

The primary objective of this pilot trial was to evaluate the safety of TEVGs as extracardiac modified Fontan conduits in patients with single ventricle cardiac anomalies. The secondary objective was to determine the growth potential of the TEVG by evaluating its change in length between 6 months and 3 years after implantation. The original design was to enroll six patients and monitor them with serial echocardiography and MRI over a three-year period.

*Growth analysis*

The growth capacity of the TEVG was evaluated using serial MRI studies performed 6 months and 3 years after implantation. The growth capacity of the TEVG was estimated by comparing its change in length over time against the patient's Glenn shunt SVC measured from its first branch to the pulmonary artery anastomoses.

*Safety analysis*

Patients were seen and evaluated by the study team during the initial hospitalization and following TEVG implantation at all scheduled follow up appointments (1, 6, 12, 24, and 36 months postoperatively), as well as at any unscheduled cardiology or cardiac surgery appointments or hospitalizations. In addition, the study nurse contacted the patient's parent or guardian monthly by phone to review their medical status using a standardized survey. All adverse events were recorded and their grade and attribution were determined by the study team and reviewed with the data safety monitoring board. The data were analyzed and compared to the incidence of graft-related complications in the initial pilot study performed at Tokyo Women's Hospital in Japan.

*Angioplasty*

Patients who developed stenosis defined as >50% reduction in luminal diameter of the TEVG were treated with angioplasty without stenting.

**Computational Model**

*Constrained mixture framework*

The $\alpha$ = 1,..., n structurally significant constituents are endowed with individual time-varying rates of production and degradation as well as material properties, but are constrained to deform with the graft as a whole. Hence, constituent-specific deformation gradients at a current growth and remodeling time s for material produced at an intermediate time $\tau$

are

$$\mathbf{F}^{\alpha}_{n(\tau)} = \mathbf{F}(s)\mathbf{F}^{-1}(\tau)\mathbf{G}^{\alpha}(\tau)\,,$$

where $\mathbf{F}$ is the deformation gradient for the bulk material (i.e., mixture) and $\mathbf{G}^{\alpha}$ are constituent-specific "deposition stretches" at which new matrix is incorporated within external material, whether polymer or extracellular matrix. The $(\tau)$ denotes the natural configuration at which the individual constituents are stress-free. A simple rule of mixtures expression for the elastic energy that is stored in the graft upon deformation stems from the sum of the constituent-specific stored energies, namely $W = \sum W^{\alpha}$, which allows a classical continuum formulation of the wall mechanics, with the Cauchy stress $\mathbf{t} = 2\mathbf{F}\,(\partial W/\partial \mathbf{C})\,\mathbf{F}^{T}/\det(\mathbf{F})$ where $\mathbf{C} = \mathbf{F}^{T}\mathbf{F}$ is the right Cauchy-Green tensor. Growth and remodeling processes are slow and thus can be described via a series of quasi-static equilibrium states such that $\mathrm{div}\,\mathbf{t} = \mathbf{0}$ satisfies Newton's second law of motion for a continuum body. Because each constituent can evolve, the stored energy density is taken to be

$$\rho(s)W^{\alpha}(s) = \rho^{\alpha}(0)Q^{\alpha}(s)\widehat{W}^{\alpha}\left(\mathbf{F}^{\alpha}_{n(0)}(s)\right) + \int_{0}^{s} m^{\alpha}(\tau)q^{\alpha}(s,\tau)\widehat{W}^{\alpha}\left(\mathbf{F}^{\alpha}_{n(\tau)}(s)\right)d\tau \quad \text{where } \rho(s) \text{ is the}$$

overall mass density, $\rho^{\alpha}(0)$ the initial apparent mass density of constituent $\alpha$, $\widehat{W}^{\alpha}\left(\mathbf{F}^{\alpha}_{n(0)}(s)\right) > 0$ stored energy density of the material that was initially present in the scaffold, $m^{\alpha}(\tau) > 0$ the mass density production rate, $q^{\alpha}(s,\tau) \in [0,1]$ the survival fraction of material produced at time $\tau$ that remains at time s, and $\widehat{W}^{\alpha}\left(\mathbf{F}^{\alpha}_{n(\tau)}(s)\right) > 0$ the energy stored at time s in the cohort of constituent $\alpha$ that was produced at time $\tau \in [0, s]$ and deformed via $\mathbf{F}^{\alpha}_{n(\tau)}$. The deposition and degradation of cellular and matrix components are governed according to immunological and mechanical stimuli, with production and degradation rates increased for inflammation-driven turnover. A specific functional form is given in Results.

*Computational studies*

The computational model was first used to predict the evolving normalized luminal diameter and wall thickness of an implanted clinical TEVG using values of the model parameters determined by fitting biomechanical and geometric data from mouse experiments, with appropriate modifications to geometric (diameter/thickness ratio, $D/H = 16$ for the clinical scaffold vs. $D/H = 3$ for the mouse scaffold) and microstructural (scaffold pore size $r_p = 41.9\ \mu m$ for clinical scaffold vs. $11.2\ \mu m$ for mouse scaffold) properties of the simulated TEVG to account for differences in the clinical and mouse scaffolds. Since the inflammation-driven kinetics were based on murine data and the immune response is known to play a critical role in stenosis, the effects of four key inflammatory parameters ($\delta$, $\beta$, $K^i_h$, and $K^i_{max}$) on model outputs were investigated. Parametric studies were performed by varying the value of an individual inflammatory parameter while holding the other three parameters constant and vice versa. This allowed to isolate effects of each parameter on the evolving TEVG properties, including normalized diameter, wall thickness, diameter compliance, and inflammatory areal mass density. Diameter and thickness were normalized by the original values for the implanted TEVG scaffold; normalized diameter compliance was calculated as the change in diameter for a 50% increase in pressure from the homeostatic pressure normalized by the original graft diameter. The depicted parameters (see **Table 1**) were chosen to capture a broad range of potential physiologic outcomes based on pilot simulations.

**Table 1.** Inflammatory parameters tested.

| . Inflammatory Parameters (inputs) | | | |
|---|---|---|---|
| **Parameters** | **Inflammatory Response Effects** | **Values** | **Units** |
| $\delta$ | onset and duration of inflammatory response | [0.075, 0.15, 0.30, 0.50, 0.70] | 1/Days |
| $\beta$ | shape and skew of inflammatory response | [1, 1.5, 2.0, 3.0, 4.0] | (-) |
| $K^i_h$ | rate of inflammatory cell/matrix production and degradation | [0.5, 1.0, 2.0, 4.0, 8.0] | (-) |
| $K^i_{max}$ | rate of inflammatory cell/matrix degradation | [5, 10, 20, 40, 80] | (-) |

The key equation within the overall computational model of TEVG development that accounts for changes in mass via changes in the rates of production $m^\alpha$ and removal $q^\alpha$ of different constituents $\alpha$, each of which can depend on the inflammatory burden (superscript $i$), due to the foreign body response, and mechanobiological responses (superscript m), due to deviations $\Delta$ in circumferential wall stress $t_\Theta$ and luminal wall shear stress $\tau_w$ from homeostatic target values. Four key model parameters and possible ranges therein were identified from prior experiments in immuno-competent and immuno-compromised mice to bound the possible inflammation-driven process of neovessel formation.

**Evolving Mass Turnover (due to production $m^\alpha(\tau)$ and removal $q^\alpha(s,\tau)$)**

$$\rho(s) \text{ (graft mass)} = \rho^p(s) \text{ (polymer mass)} + \rho^i(s) \text{ (immuno-mediated mass)} + \rho^m(s) \text{ (mechano-mediated mass)}$$

with $\quad \rho^\alpha(s) = \rho^\alpha(0)Q^\alpha(s) + \int_0^s m^\alpha(\tau)q^\alpha(s,\tau)d\tau$ , for $Q^\alpha(s) \in [0, 1]$, $\alpha = p$, $i$, or m where

$m^i(\tau) = f(\delta, \beta, K_h^i)$ and $q^i(s,\tau) = g(K_h^i, K_{max}^i)$ for immuno-mediated constituents and $m^m(\tau) = \hat{f}(\Delta t_\theta, \Delta\tau_w)$ and $q^m(s,\tau) = \hat{g}(\Delta t_\theta)$ for mechano-mediated constituents

*Large animal study*

*Study design*

The objective of this study was to test a computationally generated output, that is, to quantify the natural history of neotissue formation and thus neovessel development over 1 year in an established IVC interposition TEVG model. Seeded TEVGs were implanted in 24 lambs, and *in vivo* data were collected via serial angiography and intravascular ultrasound at 1 week, 6 weeks, 6 months, and 1 year. The 1-week time point was used for baseline anatomic information; it provided comparable data to the immediate post-operative period, but allowed the animal to recover from the initial surgical insult and decreased the risk associated with the prolonged anesthesia needed to perform the implantation surgery and an initial catheterization during the same period. Pilot data demonstrated significant stenosis at 6 weeks, consistent with computational predictions, and the later times were chosen to monitor graft performance over the long term. The primary endpoint was the narrowest cross-sectional area of the graft on intravascular ultrasound imaging at each time. No data were excluded from the study.

*Large animal scaffold*

TEVG scaffolds were provided by Gunze Ltd. (Tokyo, Japan) and were identical to those used in clinical trial: knitted PGA core with a 50:50 co-polymer sealant of PCLA.

*Bone marrow aspiration, assembly, and implantation of the TEVG* Twenty-four juvenile lambs underwent bone marrow aspiration (5 mL/kg body weight) and implantation of an autologous cell-seeded TEVG as intrathoracic IVC interposition grafts. Animals were anesthetized using propofol (5 mg/kg) for induction and isoflurane (1-4%) or propofol (20-40 mg/kg/hr) for maintenance. Lambs were placed in the lateral recumbent position, and the area overlying the iliac crest was shaved and prepped in standard sterile fashion. A 2-mm incision was made and an aspiration needle was inserted into the bone. Heparinized syringes (100 U/mL) were used to aspirate 5 mL/kg of bone marrow.

Following aspiration, the bone marrow was processed using Ficoll density gradient separation to isolate the bone marrow-derived mononuclear cells as previously described. Briefly, bone marrow was filtered through 100-$\mu$m cell strainers to remove bone spicules and clots. A 1:1 dilution was achieved with phosphate buffered saline (PBS) and the bone marrow was layered onto Ficoll 1077 (Sigma-Aldrich, St. Louis, MO). The plasma and mononuclear cell layers were isolated after centrifugation. The mononuclear cell layer underwent two washes with PBS to yield a cell pellet that was diluted in 20 mL of PBS to seed the scaffold. The mononuclear cells were vacuum-seeded onto the scaffold, which was incubated in plasma until the time of implantation.

The scaffolds were implanted in the intrathoracic IVC as previously described. Lambs were placed in a left lateral recumbent position. Depending on each animal's anatomy, a right thoracotomy was made in the fifth or sixth intercostal space, and the thoracic IVC was dissected between the diaphragm and right atrium. A cavoatrial shunt was placed to maintain perfusion during cross-clamping of the IVC. The vessel was clamped and a 2-cm segment of a diameter-matched seeded scaffold was implanted, with end-to-end anastomoses performed using a running nonabsorbable monofilament suture. No native vessel was removed. Titanium vascular clips were applied to the suture tails to mark the anastomoses for postoperative imaging. The chest wall, overlying muscle, and skin layers were reapproximated with absorbable sutures.

*Interventional imaging*

Postoperative catheterizations were performed at 1 week, 6 weeks, 6 months, and 1 year. Additional imaging was performed as needed based on the animals' clinical conditions. After sedation and intubation, lambs were placed in a left lateral recumbent position. The right internal jugular vein was cannulated and a 9-French sheath (Terumo, Somerset, NJ) was inserted followed by an intravenous bolus of heparin (150 U/kg). A 5-French JR 2.5 catheter (Cook Medical, Bloomington, IN) was passed into the right internal jugular vein through the SVC and into the right atrium. Using an angled Glidewire (Terumo), the JR catheter was then passed through the TEVG into the intraabdominal IVC where a Rosen exchange guidewire (Cook Medical, Bloomington, IN) was placed. The JR catheter was then exchanged for a 5-French multi-track angiographic catheter (NuMed, Hopkinton, NY) which was used to measure hemodynamic pressures in the intraabdominal IVC, intrathoracic IVC below and above the TEVG, and within the TEVG. A mean pressure gradient was calculated by subtracting the mean pressure above the TEVG from the mean pressure below the TEVG. A digital angiogram was then obtained by injecting ioversol 68% (Mallinckrodt Pharmaceuticals, Raleigh, NC) through the multi-track angiographic catheter positioned in the intraabdominal IVC. Diameters were measured at seven points: the intraabdominal IVC, low intrathoracic IVC (on the diaphragmatic side of the TEVG), proximal anastomosis (defined with respect to blood flow), midgraft, distal anastomosis, high intrathoracic IVC (on the atrial side of the TEVG), and the area of most severe narrowing. The proximal and distal anastomoses were identified by the aforementioned surgically-placed radiopaque clips. A 0.035-inch digital intravascular ultrasound catheter (Volcano, San Diego, CA) was advanced through the graft over the Rosen guidewire. This was used to obtain images at the same seven points measured during angiography. These images were analyzed using Volcano software to obtain a cross-sectional area as described previously.

Due to the requirement to size match grafts at implant, angiographic and IVUS data were normalized to their respective 1-week measurement and graft stenosis was thus represented as a fold change relative to the baseline midgraft size at 1 week, namely

$$\left(\frac{measured\ value - 1wk\ midgraft\ value}{1wk\ midgraft\ value}\right)$$

Neotissue development within the TEVG was measured using intravascular ultrasound and reported as a percentage of the diameter of the TEVG. Graphical reconstructions of IVUS imaging data were performed using Rhino 3D (Seattle, WA).

*Euthanasia*

At the prescribed endpoint, animals were deeply sedated with ketamine (20 mg/kg) and diazepam (0.02-0.08 mg/kg), followed by induction of bilateral pneumothoraces and exsanguination. A complete veterinary necropsy was performed at the time of TEVG explantation. Animals were also euthanized if they developed critical stenosis, defined here as graft narrowing with systemic symptoms. Animals that were not euthanized for critical stenosis were euthanized at 6 months (n=5) and 12 months (n=2) post implantation. The remaining animals were survived for long-term follow up, with three euthanized at 18 months for late-term mechanical testing.

**Neotissue structural characterization**

*Histology and immunohistochemistry*

TEVG explants were fixed with 4% formalin, dehydrated, and embedded in paraffin before 4-$\mu$m transverse sections of the midgraft were prepared and mounted on slides and heat fixed. Standard techniques were adopted for hematoxylin & eosin and Picro-Sirius Red staining. Immunohistochemistry was used to detect macrophage antigen CD68 (CD68, 1:500, Abcam), and alpha smooth muscle actin (aSMA, 1:2000, Dako). Samples underwent heat-induced antigen retrieval with Dako target retrieval solution (90°C, pH 6.0) followed by blocking endogenous peroxidase activity (0.3% H2O2 in H2O) and non-specific binding (3% normal goat serum in Background Sniper, BioCare Medical). After primary antibody incubation, sections were incubated sequentially in appropriate biotinylated secondary antibodies (1:1500, Vector) and streptavidin-horseradish peroxidase (Vector). DAB+ substrate chromogen (Vector) was used for color development. All samples were counterstained with Gill's hematoxylin (Vector) prior to dehydration and coverslipping.

*Image quantification*

Photomicrographs of histological stains were quantified using ImageJ. For Picro-Sirius Red stained specimens, tiled 25x magnification images were used to capture the entire vessel area. Areas of interest were quantified using the Color Threshold command. For the immunohistochemically stained slides, four representative tiled images were taken at 100x around the vessel, each capturing the full width of a section of graft or native IVC. The images were assessed with use of the Color Deconvolution and Threshold commands to quantify positively stained area or count positive cells.

*Biaxial mechanical testing*

A composite specimen, including the TEVG and adjacent proximal and distal thoracic IVC, was excised at 18 months post-implantation in the lambs from right atrium to the diaphragm (59.3 ± 16.2 mm), then cleaned of perivascular tissue by blunt dissection. The specimens were cannulated on custom acrylic cannulae, mounted within a custom computer-controlled testing device, and immersed in Hank's buffered physiologic solution at room temperature. Outer diameter was measured using a video camera and length was prescribed via a stepper motor; luminal pressure and axial force were measured using standard transducers. The specimen was equilibrated for 15 minutes under low flow at a pressure of 5 mmHg, then preconditioned with 6 cycles of pressurization (1 to 30 mmHg), both at a fixed value of the *in vivo* axial stretch. Acquisition of passive data consisted of cyclic pressure-diameter tests (1.5 to 30 mmHg) at three different fixed values of axial stretch (95%, 100%, and 105% of the *in vivo* value). Data from the unloading curve of each protocol were used for analysis.

*Computational model validation*

After gathering data on the evolving ovine TEVG, and noting differences between the predicted (based on parameters from the prior mouse studies) and measured evolving geometries, a non-intrusive optimization technique was used, the Surrogate Management Framework described in detail previously, to identify values of the four key inflammatory parameters to capture the evolving normalized luminal diameter and wall thickness of the lamb grafts throughout the first year of implantation as measured by intravascular ultrasound. As the intravascular ultrasound showed non-circular graft cross-sections, the hydraulic diameter of the TEVGs was calculated from area measurements for the fitting process. Bounds for the Surrogate Management Framework optimization were informed by the parametric studies. To validate the computational model, the measured compliance of the TEVG at 18 months with that predicted from the Growth and Remodeling model were compared.

*Statistical analysis*

Statistical analyses were performed and graphs created using GraphPad Prism version 7.03 (GraphPad Software, Inc., La Jolla, CA). Comparison between the incidence of early stenosis in the Japanese vs United States clinical trials was performed via two-tailed Fisher's exact test. Serial measurements from the ovine study (angiography and IVUS) were first normalized to paired 1-week values to control for variable scaffold sizes used at implantation to ensure size matching to the native vessel or variable degrees of anastomotic narrowing as a result of the implantation procedure and are thus represented as a fold change relative to the respective 1-week measurement. Pressure measurement or normalized angiographic and IVUS values were analyzed using an ordinary oneway ANOVA with Tukey's post-hoc multiple comparison's test. Histomorphometric and micrographic data (wall thickness, aSMA+ area fraction, CD68+ cells/mm$^2$, and collagen area fraction) were analyzed via ordinary one-way ANOVA with Tukey's post-hoc multiple comparison's test. For all statistical tests, $\alpha$ was restricted to 0.05 and *p* values < 0.05 were considered statistically significant.

### *Ethical compliance*

*Clinical trial*

Institutional review board approval was obtained from Yale University (HIC #0701002198 and Nationwide Children's Hospital (IRB12-00357). This clinical trial was performed under FDA IDE 14127 in compliance with good clinical practice guidelines.

*Ovine study*

The Institutional Animal Care and Use Committee of Nationwide Children's Hospital (Columbus, OH) reviewed and approved the protocol (Ar13-00079). Representatives of the animal care staff monitored all animals intraoperatively and during their postoperative courses. Animal care was within the humane guidelines published by the Public Health Service, National Institutes of Health (Bethesda, MD) in the Care and Use of Laboratory Animals (2011), as well as within USDA regulations set forth in the Animal Welfare Act.

## Results

### *Design and characterization of the TEVG*

TEVGs were assembled by seeding autologous bone marrow-derived mononuclear cells onto a biodegradable tubular scaffold. The scaffolds (Gunze Ltd, Kyoto, Japan) were made from poly(glycolic acid) fibers (PGA) and a copolymer of caprolactone and lactide (PCLA) synthesized by ring opening polymerization with a 50:50 molar composition. The PGA fibers were knitted into a tube and coated on the inner and outer surface with the PCLA solution and then freeze dried under a vacuum, creating a matrix of knitted PGA fibers embedded within a porous sponge of PCLA.

The scaffold was designed to degrade by hydrolysis over approximately 6 months. Scaffold dimensions measured either $16 \pm 0.5$ mm or $18 \pm 0.5$ mm on the inner diameter, $13 \pm 0.5$ cm in length, and $0.7 \pm 0.1$ mm in wall thickness (Figure 1A). Quantitative scanning electron microscopy of the inner surface revealed an average pore size of $41.9 \pm 2.7$ $\mu$m and porosity of $0.87 \pm 0.01$. On the outer surface, average pore size was $36.4 \pm 6.6$ $\mu$m and porosity was $0.86 \pm 0.02$. The PGA fiber bundles were knit in a weft pattern, with a 1 mm separation between layers axially and 1 mm between peaks running circumferentially. The average PGA fiber diameter measured $15.8 \pm 1.0$ $\mu$m. TEVGs were assembled in compliance with Good Manufacturing Practice (GMP) regulations. Bone marrow (5 ml/kg body weight) was harvested on the day of surgery and the mononuclear cell fraction separated using density centrifugation in Ficoll. In the United States clinical trial, this procedure yielded an average of $20.6 \times 10^6$ (range $19.0\text{-}22.2 \times 10^6$) mononuclear cells with an average cell viability of 92.6% (range 86.5-96.8%). Flow cytometry demonstrated that 78.3% (range 73.2-85.3%) of these cells were CD45+. These cells were then seeded onto the polymeric scaffold using a custom vacuum system, which yielded an average seeding efficiency of 42.7% (range 23-61.4%). The seeded scaffold was incubated in autologous plasma (obtained from the non-mononuclear cell fraction after density gradient centrifugation) for two hours prior to implantation as a vascular scaffold on the same day the TEVG was assembled. All TEVGs used in the clinical trial met the release and post-process monitoring criteria **(Table 2).**

**Table 2.** Release Testing and Post-Process Monitoring Criteria for TEVG.

| Release Criteria | | Post Process Monitoring Criteria | |
|---|---|---|---|
| *Parameter* | *Criteria* | *Parameter* | *Criteria* |
| Viability | >70% | Anaerobic Culture | Negative |
| Manual Cell Count | >10○/ml | Aerobic Culture | Negative |
| Automatic Cell Count | >10○/ml | Fungal Culture | Negative |
| Hematocrit | <5% | CD45 | > 10○ cells/mL |
| Gram Stain | Negative | Viability | > 70% |
| Endotoxin | <20EU/Scaffold | Cell Attachment | >1000/mm$^2$ |
| Seeding Efficiency | >10% | | |

### *Clinical performance of the TEVG*

The FDA-approved clinical trial evaluated the safety and growth capacity of the TEVG when used as a vascular conduit connecting the inferior vena cava (IVC) to the pulmonary artery in children with single ventricle cardiac anomalies undergoing a modified Fontan operation (Figure 12 and Table 3).

**Table 3.** Clinical trial patient demographics

| Patient # | Age (yrs) | Sex (M/F) | Ethnicity | Diagnosis |
|---|---|---|---|---|
| 1 | 3 | F | Hispanic | Single ventricle LV |
| 2 | 2 | M | Caucasian | Single ventricle RV |
| 3 | 2 | F | Caucasian | Single ventricle LV |
| 4 | 2 | F | Caucasian | Single ventricle LV |

Growth capacity was assessed using serial magnetic resonance imaging (MRI) studies, performed 6 months and 3 years after implantation. Noting that the IVC is a capacitance vessel that changes its diameter on a moment-to-moment basis, the growth of the TEVG by comparing its change in length over time against that of an internal control was estimated:

the superior vena cava (SVC) when anastomosed to the pulmonary artery (called a Glenn shunt, which is a component of the Fontan operation). The four TEVGs increased 2.5 mm (range 1.1 to 4.2 mm) in length between 6 months and 3 years after implantation whereas the Glenn shunt increased 1.5 mm (range 0.9-2.4 mm) in length during the same period. The average percent increase in length of the TEVG and Glenn shunt were both 7%.

Safety analyses demonstrated no graft-related deaths, catastrophic graft failures, or complications requiring graft replacement during the 3-year study. All four patients continue to do well 4-7 years after implantation. However, three of the four patients developed critical stenosis (> 50% narrowing of the graft diameter) and were successfully treated with angioplasty 5-8 months after implantation. There were no additional graft-related complications. Enrollment was capped at 4 patients instead of the intended 6 due to this unexpectedly high incidence of early TEVG stenosis: 3 out of 4 patients (75%) in the United States trial developed stenosis and were treated with angioplasty, while only 1 out of 25 (4%) in the original Japanese trial developed stenosis and required angioplasty within three years after implantation (two-sided Fisher's exact test, p<0.01).

Figure 12 demonstrates a flow diagram for a pilot study investigating the clinical use of tissue engineered vascular grafts in congenital heart surgery. The study **objectives** were as follows. The primary objective of this pilot study was to determine the safety of using tissue engineered vascular grafts as large diameter, high-flow, low-pressure conduits in pediatric patients requiring extracardiac total cavopulmonary connection (EC TCPC) for palliative treatment of single ventricle cardiac anomalies. The secondary objective was to determine the growth potential of the tissue engineered vascular grafts using serial magnetic resonance angiography. The **Trial Design** was as follows. This investigation was a prospective, nonrandomized Phase 1 clinical trial determining the safety of the use of tissue engineered vascular grafts as conduits for EC TCPC. **Eligibility Criteria:** All patients who were considered candidates to undergo EC TCPC for completion of a modified Fontan for palliation of their congenital cardiac anomaly during the course of the investigation at the institution were considered for enrollment in the trial. No patient was excluded based on age, gender, or ethnicity. Inclusion criteria included patients with single ventricle anomalies who were candidates for EC TCPC, who volunteered, and provided informed consent. **Exclusion Criteria:** Exclusion criteria included urgent/emergent operative status, major chromosomal anomalies, the need for a pacemaker, pulmonary vascular resistance greater than 4 um$^2_2$ (u= Wood's units), abnormal venous drainage (interrupted IVC), presence of moderate to severe atrio-venticular valve regurgitation, or other significant medical problems. History of any other condition or other significant medical problems that, in the opinion of the investigator, precluded compliance with protocol-specified procedures. **Study Sites:** This single institutional study was initiated at Yale-New Haven Hospital but then transferred to Nationwide Children's Hospital. The data housing facility is on site (Nationwide Children's Hospital, Columbus, OH). **Outcome Measures:** Primary end points of the study included determination of graft failure rates and graft related morbidity and mortality. Graft failure was defined as any graft narrowing/occlusion or dilation/rupture requiring surgical or endovascular intervention. Graft related morbidity and mortality included any post-operative complication such as any thromboembolic or infectious event that required treatment and was thought likely to be caused by the tissue engineered vascular graft as determined by the investigators and confirmed by the data safety monitoring board. **Sample Size:** an initial plan was for enrolling a total of 6 patients. Since a higher incidence of stenosis was experienced than expected, the study was concluded at 4 patients total. Since this was a pilot study to look at rates of adverse events among patients who receive these vascular grafts, the study was not powered for testing. **Study Dates:** FDA approval received in December 2009 while at Yale New Haven Children's Hospital associated with Yale University, implanted the first patient in August 2011. The study was relocated to Nationwide Children's Hospital associated with The Ohio State University in September 2012. After completion of required inspection of the institutional facility, the enrollment was resumed in March 2014 with a completion of all clinical follow-ups in August 2017. **Study Termination:** A higher incidence of TEVG stenosis was experienced in the clinical trial than predicted based on the original pilot study in Japan. Based on these findings the study was voluntarily placed on hold and requested a closed DSMB meeting. The DSMB has subsequently met and recommended temporarily putting the study on hold and re-evaluating the data. **Trial Registration:** NCT01034007.

### *Building a computational model of neovessel development*

A general constrained mixture theory was used for describing changes in mass and changes in the microstructure of soft tissues as the basis for the model. The computational model to simulate the evolving geometry, composition, and mechanical properties of TEVGs implanted in mice over time was previously developed. The model considered the degrading polymeric scaffold, organizing vascular neotissue, and newly produced collagendominated extracellular matrix to be separate structurally significant constituents. Specifically, datadriven constitutive relationships define the intrinsic material properties for each of the $\alpha$ = 1,2,...$n$ constituents as well as their individual rates of production and removal: a neoHookean relation describes the mechanical behavior of the polymer, a Fung-exponential relation describes that of the neotissue, the rate of mass density production m)$\tau$ > 0 depends on mechanobiological stimuli, such as intramural wall stress, and immunobiological stimuli, proportional to macrophage invasion, and removal is defined via a survival function q) s, $\tau \in [0,1]$ that tracks the percentage of the material produced at time $\tau \in [0, s]$ that remains at time s. For example, deviations in intramural stress from homeostatic conditions regulate mechano-mediated kinetics, as vascular cells typically promote mechanobiological homeostasis, while scaffold microstructure, as quantified from scanning electron microscopy, modulates inflammation-driven kinetics since a pore size sufficient for cellular infiltration is a critical regulator of macrophage activity and phenotype.

Previous studies of immuno-competent and immuno-compromised mice identified four key model parameters that control the deposition and degradation of inflammation-driven extracellular matrix (**Table 1**): $\delta$ modulates the onset and duration of the inflammatory response, $\beta$ controls the skewness of the production function, $K^i_h$ controls rates of inflammatory extracellular matrix production and degradation, and $K^i_{max}$ scales the inflammatory effects of extracellular matrix degradation, with production given by

$$m^\alpha_{infl}(\tau) = m^{\alpha,infl}_h (1 - \exp(-\tau)) K^i(\tau)\delta^\beta \tau^{\beta-1} \exp(-\delta\tau),$$

Where $m^{\alpha,infl}_h$ is the basal rate of production of matrix constituent $\alpha$ driven by inflammation and

$K^i(\tau) = K^i_h\left(r_p(\tau)/r_n\right) + K^i_w$ , is the gain on a gamma distribution function that describes the onset and resolution of the foreign body response to the degrading polymer, with $r_p(\tau)/r_n$ the pore size of the scaffold normalized by a critical pore size for cellular infiltration, and $K^i_w$ a basal gain on inflammation-driven extracellular matrix production. Degradation of the inflammatory extracellular matrix was governed by a first-order kinetic type decay,

$$q^\alpha(s,\tau) = \exp\left(-\int_\tau^s k^i_h(1 + K^i(t)/K^i_{max})\,dt\right),$$

where $k^i_h$ is a rate-parameter and $K^i_{max}$ is again the maximum value of $K^i(\tau)$.

*Parametric simulations of neotissue formation*

Myriad simulations of neovessel development **(Figure 7A)** resulted by varying the value of an individual inflammatory parameter while holding fixed the other parameters **(Table 1).** This allowed accomplishing something not possible with *in vivo* studies - to isolate effects of individual contributors to the evolution of key clinical parameters, including TEVG inner radius, wall thickness, diameter compliance, and neotissue accumulation over the desired time course. The depicted parameter ranges captured a broad range of potential physiologic outcomes based on broader preliminary studies (not shown). Overall, the simulations predicted findings similar to those suggested by the clinical trial, namely an early narrowing of the TEVG due to increased inflammation-driven neotissue thickening of the wall and narrowing of the lumen. Interestingly, however, the model also predicted that such narrowing would reverse spontaneously for a broad range of cases as the immune response waned with polymer degradation and subsequent mechanomediated neotissue degradation outpaced deposition as wall stress dropped well below normal homeostatic values due to both the narrowing and thickening **(Figures 7A-7H).** The prospect that TEVG stenosis could reverse spontaneously had not been previously considered. The computational model thus generated an unexpected outcome that was tested experimentally using a large animal model.

In Figures 7A-7H, each plot focuses on the early evolution, up to 52 weeks post-implantation, of normalized luminal diameter (top row) and wall thickness (bottom row) for variations in $\delta$, $\beta$, $K^i_h$, and $K^i_{max}$, with arrows showing the direction of increasing values. The predicted transient reduction in luminal diameter and subsequent recovery is shown, with these changes resulting in part from a thickening of the wall that encroaches on the lumen within an initially stiff polymeric scaffold that subsequently degrades, thus decreasing immuno-control while increasing mechano-control of the biology and mechanics.

*TEVG stenosis reverses spontaneously in a large animal model*

The predictions of the computational model were tested by performing a time-course study in an ovine intrathoracic IVC interposition graft model. The ovine IVC interposition graft is a validated model that is used as a surrogate for the Fontan operation since there are no large animal models with single ventricle cardiac anomalies and performance of a Fontan operation on an animal with a structurally normal heart is associated with excessive mortality (>80%). Size-matched TEVGs were implanted into 24 juvenile lambs **(Figures 13A and 13B: Ovine Study Design and Surgical Outcomes).** TEVG morphology was monitored serially over a 12- to 18-month period in all surviving lambs using angiography and intravascular ultrasound to measure luminal diameter and cross-sectional area. Angiography revealed significant TEVG stenosis at 6 weeks (-0.48 ±0.16-fold diameter change from 1 week, one-way ANOVA with Tukey's multiple comparisons test: a=0.05, $p < 0.0001$). As predicted, the stenosis improved spontaneously by 6 months without endovascular intervention and all TEVGs remained patent at 1 year and beyond.

Intravascular ultrasound suggested that early TEVG narrowing resulted from appositional growth of neotissue on the luminal surface of the scaffold, thus thickening the wall and narrowing the lumen. At 6 weeks, the TEVG stenosis was localized to the mid-distal segment of the graft (i.e., region closer to the heart) while no changes were appreciated at the proximal anastomosis. Quantitative intravascular ultrasound assessment confirmed that the luminal area decreased significantly from 1 to 6 weeks (-0.67 ±0.17-fold area change from 1 week midgraft area, one-way ANOVA with Tukey's multiple comparisons test: a= 0.05, $p < 0.0001$), the narrowing reversed by 6 months, and grafts remained patent at 1 year **(Figure 8A).** During the same period, the wall reached its maximum thickness at 6 weeks (range 1.5 to 4.6 mm, one-way ANOVA with Tukey's multiple comparison's test: a= 0.05, $p < 0.0001$) and then progressively thinned over the 1 year time course **(Figure 8B).**

Hemodynamic data during each angiography was also collected. The mean pressure gradient across the graft followed the aforementioned morphometric changes. The gradient increased significantly from 1 to 6 weeks ($0.5 \pm 0.5$ vs. $11.8 \pm 5.5$ mmHg, $p < 0.001$), then decreased to near baseline by 6 months ($1.3 \pm 2.8$ mmHg, one-way ANOVA with Tukey's multiple comparisons test: a= 0.05, $p < 0.001$ vs. 6 weeks, $p = 0.8883$ vs. 1 week) **(Figures 14A-14D: Angiography, IVUS, and Hemodynamic Data Analysis).** The largest pressure gradients tended to correspond to the most narrowed grafts. Two of 22 animals (9.1%) that developed stenosis also developed symptoms including ascites, lethargy, and weight loss. Both of these lambs had mean pressure gradients > 19 mmHg at 6 weeks, and their grafts were two of the three most narrowed on angiography, both measuring less than 4 mm luminal diameter at the narrowest point. These two symptomatic animals were euthanized. Despite the formation of significant TEVG stenosis in all animals, the stenosis resolved in the remaining lambs, which remained asymptomatic. None of the grafts acutely occluded and no animals died as a result of stenosis.

### *Computational model validation*

Whereas the parametric studies were parameterized based on studies of a murine IVC-interposition TEVG that consisted of a similar scaffold design as used in the ovine and clinical grafts, it was tested whether the model could also describe the *in vivo* ovine data. These data fit well for all times (R2 = 0.83) using many model parameters from the murine experiments (e.g., mechanical properties of collagen and basal rates of collagen turnover), but generated ovine-specific values for the four key parameters that control the temporal inflammatory response: $\delta$ = 0.32 days-1, $\beta$ = 3.96, $K^i_h$ = 5.13, and $K^i_{max}$ = 72. These best-fit values of the parameters were identified using a Surrogate Management Framework optimization method, as in prior growth and remodeling studies of native blood vessels. Among the different computed metrics, note in particular the evolution of luminal diameter and wall thickness which dictate the presence or absence of stenosis **(Figures 8A and 8B).**

### *Evolving cellular composition of neovessel is consistent with computational model predictions*

The changes in the ovine neotissue over time were characterized using histology and immunohistochemistry. Histological sections confirmed changes in the lumen and wall of the TEVG that were observed with *in vivo* imaging, and verified that the transient luminal narrowing was primarily due to scaffold thickening and partly due to neotissue formation on the luminal surface of the scaffold that appeared to resolve by 6 months after implantation. Quantitative histomorphometry demonstrated that wall thickening peaked 6 weeks after implantation, consistent with the computational predictions **(Figures 9A and 9B).** Immunohistochemical staining for a-smooth muscle actin (aSMA) identified some positive cells along the luminal surface of the scaffold as well as within the scaffold; their number were greatest at 6 weeks. The aSMA+ cells that form along the luminal surface were presumed to be smooth muscle cells that proliferated and contributed to the neotissue that caused TEVG stenosis. The overall number of aSMA+ cells peaked early, which fit well with the predicted early exuberant production of matrix by the computational model **(Figures 9C and 9D).** The degree of macrophage infiltration was assessed using immunohistochemical staining against CD68. Quantitative immunohistochemistry revealed the greatest macrophage density at 6 weeks. Beyond 6 months, the number of macrophages diminished consistent with scaffold degradation, which also fit well with the computational predictions **(Figures 9E and 9F).** Taken together, these findings show that the formation of TEVG stenosis is largely inflammation driven.

### *Computational model accurately predicts neovessel biomechanics*

The sum of the mechanical contributions of the polymeric scaffold and neotissue (which is primarily determined by the extracellular matrix component of the neotissue) determine the biomechanical properties of the TEVG. The scaffold degradation and neotissue formation was characterized using polarized light images of Picro-Sirius Red (PSR) stained sections from ovine TEVGs obtained 1 week, 6 weeks, 6 months, and 1 year after implantation, which revealed both the degradation of PGA fibers and the deposition and maturation of collagen fibers. The PGA fibers remained highly organized within the scaffold 1 week after implantation, but had thinned and begun to show evidence of early fragmentation at 6 weeks. Only rare thin individual fragments of the PGA fibers were visible by 6 months after implantation and beyond. In contrast, minimal staining was detected for fibrillar collagen at 1 week. The total amount of collagen in the neotissue peaked 6 weeks after implantation, with significant amounts of thin (green) fibers within the scaffold and along its luminal surface. Collagen density increased steadily over the first year as it compacted and matured **(Figures 10A and 10B).** This collagen appeared to be the primary extracellular matrix component of the neotissue that was associated with TEVG stenosis. The collagen also appeared to remodel between 6 weeks and 6 months, as the scaffold degraded and the wall thinned; at 6 months, collagen fibers appeared orange, signifying they were of medium thickness. By one year after implantation, the wall became even thinner and the collagen fibers appeared thicker (red) and denser, suggestive of normal, mature vascular collagen.

*In vitro* biaxial mechanical testing of TEVGs excised 1.5 years post-implantation revealed a structural response (pressure-diameter) of the TEVG that was similar to model predictions **(Figure 10C).** The final compliance of the TEVG, which was predicted by the computational model as a validation step rather than based on a fit to data, also matched well with values found from the *in vitro* mechanical characterizations **(Figure 10D).** Importantly, the associated values of the material parameters in the computational model suggested that this bulk behavior resulted from a higher inflammation-driven matrix turnover than mechano-mediated matrix turnover throughout much of neovessel development, as the mechanical stimuli for neotissue production were attenuated by the high wall thickness and low diameter.

**Discussion**

The first FDA-approved clinical trial evaluating the use of TEVGs in the repair of complex congenital cardiac anomalies confirmed that stenosis is the most prevalent graft-related complication, but formation of early TEVG stenosis occurred at a much higher incidence than previously observed.

To gain mechanistic insight into the complex immunological and mechanical processes underlying the formation of early TEVG stenosis, a computational model of neovessel development was used to study parametrically the relative contributions of multiple critical factors that control neotissue formation. This model predicted spontaneous resolution of stenosis, a finding that was reproducibly confirmed in an ovine IVC interposition graft model. Asymptomatic TEVG stenosis in the lambs could be monitored safely without acute graft failures or thrombosis over the entire 1 to 1.5-year study period. Based on the available data, symptoms and the mean pressure gradient across the graft could be primary criteria in assessing the clinical significance of TEVG stenosis rather than morphometric changes alone. Because most stenoses resolved naturally, appropriate monitoring rather than overly aggressive intervention may be the way forward.

Collectively, the results from the previous studies coupled with the human clinical trials, computational simulations, and ovine studies suggest that implantation of a cell-seeded PGA/PCLA scaffold as a TEVG within the vasculature sets into motion an inflammation-driven, mechanomediated evolution of a neovessel over time. The polymer initially incites a strong foreign body response and host inflammatory cells infiltrate the scaffold. At the same time, the scaffold partially shields infiltrating vascular cells and the neotissue they deposit from the hemodynamically imposed mechanical forces until the structural integrity of the polymer is lost. Mechanobiological processes thus appear to increase as scaffold integrity diminishes, and the low wall stresses due to overthickening of the graft cause degradation to outpace subsequent deposition, resulting in a progressive resolution of the initial inflammation-driven stenosis. The consequences of inflammation are not completely negative, however; indeed, some inflammation is fundamental to early host cell recruitment and neotissue formation. Infiltrating monocytes/macrophages in particular orchestrate early neotissue formation by inducing ingrowth of host endothelial cells and smooth muscle cells from the neighboring vessel wall. Overall TEVG functionality thus requires a balanced degradation of the polymeric scaffold (primarily by hydrolysis) and appropriate deposition of neotissue. It appears that it is critical to promote, but limit, inflammation while simultaneously optimizing the timing of load transferal from the initially stiff polymeric scaffold to the cell-matrix composite that constitutes the neotissue **(Figures 11A-11C).**

Figures 11A and 11B are diagrams showing implantation of the scaffold induces a foreign body reaction and mechano-mediated neotissue remodeling. Macrophages infiltrate the scaffold. Macrophages are essential for neotissue formation, but excessive macrophage infiltration leads to stenosis. The immunobiological contribution continues to build while the polymer fragments and degrades, but begins to subside as the polymer disappears; concurrently, the smooth muscle cells respond to the mechanical stimuli of the hemodynamic environment by remodeling the extracellular matrix to alter the amount of stress shielding so as to establish mechanical homeostasis. In situations where the cells in the vascular neotissue sense low mechanical stimulation, they break down and remodel extracellular matrix to reduce stress shielding until mechanical homeostasis is reestablished.

Similarly, in situations of high mechanical stimulus, these cells produce and remodel the extracellular matrix to increase stress shielding until mechanical homeostasis is attained. The mechanobiological contribution continues to build as the stress-shielding capability of the degrading polymeric scaffold diminishes. During the first 6 months after implantation, the orchestration of neotissue formation by macrophages occurs through paracrine signaling that drives cellular migration and extracellular matrix production, while after the scaffold degrades and loses its biomechanical integrity, kinetics are mediated by the ability of vascular cells to sense and respond to their local mechanical environment via the deposition and degradation of neotissue. Note that the loss of scaffold mechanical integrity precedes its disappearance, hence an overlap in inflammatory and mechanical contributions to neotissue turnover. Importantly, the exuberant production of neotissue during the inflammation-driven period results in neotissue wall stresses well below the normal homeostatic target, thus promoting a mechano-mediated degradation of neotissue that outpaces deposition, contributing to the natural resolution of the stenosis.

In conclusion, this study highlighted the utility of combining advanced computational modeling and model-driven pre-clinical experiments in translational research. A framework for creating robust computational models that can accurately predict changes in the geometry, composition, and biomechanics of the neotissue that ultimately determine graft performance was developed. Results suggested that the early stenosis observed in the clinical trial, which resulted in the study ending prematurely, would have resolved spontaneously without angioplasty. The computational simulations predicted that the scaffold could be modified to reduce the degree and duration of narrowing by altering the scaffold design (including fiber diameter, fiber alignment, porosity and pore size) to reduce the associated inflammation and stress shielding.

**Claims**

1. A cell seeding chamber for use in a closed disposable cell seeding system, the cell seeding chamber comprising:

   a housing having a variable width and a length,
   a cap comprising an opening for a suction rod insertable into the housing, and one or more lateral fluid ports, and for securing a perforated or porous mandrel that can be positioned over the suction rod, and
   a base,
   wherein the housing has a bulge positioned between about 30% and 60% of the length of the housing forming the bulge.

2. Use of a housing for a cell seeding chamber in the production of a cell seeding chamber according claim 1,

   the housing having a variable width and a length,
   an open end to accept a cap comprising an opening for a suction rod insertable into the housing, one or more lateral fluid ports, and a perforated or porous mandrel that can be inserted into the housing and positioned over the suction rod, and
   a base,
   wherein the housing has a bulge positioned between about 30% and 60% of the length of the housing forming the bulge.

3. Use of a cap for a cell seeding chamber in the production of a cell seeding chamber according claim 1:
   the cap comprising an opening for a suction rod insertable into the housing, and one or more lateral fluid ports.

4. The cell seeding chamber of claim 1, or the use of claim 2 or 3, wherein the mandrel is a porous mandrel and has a suction rod inserted therein; and/or wherein the cell seeding chamber further comprises a graft or scaffold over the mandrel of the seeding chamber.

5. The cell seeding chamber of claim 1 or 4, or the use of any of claims 2 to 4, wherein the cell seeding chamber has a lateral vent port, and/or comprises a scaffold between the mandrel and the housing.

6. A method for seeding a graft or scaffold with cells, the method comprising:

   a) connecting the seeding chamber of any one of claims 1, 4 or 5 to a closed disposable seeding system comprising a vessel with fluid comprising blood or enriched cells,
   b) inserting a graft or scaffold over the mandrel of the seeding chamber,
   c) filling the seeding chamber with the fluid,
   d) applying negative pressure to a lower mandrel outlet and emptying the chamber to about half way,

e) inverting the chamber, and

f) applying negative pressure and emptying the chamber through an upper outlet port.

7. The method of claim 6 wherein:

(a) negative pressure is provided by a syringe, pump, or vacuum source;

(b) the graft or scaffold is the polymeric graft or scaffold of claim 4 or 5;

(c) the fluid has a volume between about 10 ml and 200 ml;

(d) the fluid has between about $10^5$ white blood cells (WBC)/ml and $10^8$ WBC/ml, preferably between about $10^6$ and $10^8$ WBC/ml, more preferably between about $10^6$ and $10^7$ WBC/ml;

(e) the graft or the scaffold has a length between 10 cm and 15 cm;

(f) seeding is complete within a time period between about 1 min and 15 min, preferably between about 1 min and 10 min, most preferably between about 1 min and 7 min;.

(g) the graft or the scaffold is seeded with cells at a substantially similar cell density along the length of the scaffold; and/or

(h) the cell density is between about 0.1 x$10^3$ cells/mm$^2$ and $10^5$ cells/mm$^2$, inclusive, along the length of the scaffold, preferably between about 0.1 x$10^3$ cells/mm$^2$ and $10^4$ cells/mm$^2$, inclusive, along the length of the scaffold, most preferably between 1 x$10^3$ cells/mm$^2$ and $10^4$ cells/mm$^2$ , inclusive, along the length of the scaffold.

**Patentansprüche**

1. Zellaussaatkammer zum Verwenden in einem geschlossenen Einweg-Zellaussaatsystem, die Zellaussaatkammer umfassend:

ein Gehäuse mit variabler Breite und einer Länge,

eine Abdeckung umfassend eine Öffnung für eine in das Gehäuse einführbare Saugstange und einer oder mehreren seitlichen Fluidöffnungen sowie zur Befestigung eines perforierten oder porösen Dorns, der über der Saugstange positioniert werden kann, und

eine Basis,

wobei das Gehäuse eine Ausbuchtung aufweist, die zwischen etwa 30 % und 60 % der Länge des Gehäuses positioniert ist, die die Ausbuchtung bildet.

2. Verwenden eines Gehäuses für eine Zellaussaatkammer bei der Herstellung einer Zellaussaatkammer nach Anspruch 1,

das Gehäuse mit variabler Breite und einer Länge,

ein offenes Ende zur Aufnahme einer Abdeckung, umfassend eine Öffnung für eine in das Gehäuse einführbare Saugstange, einen oder mehrere seitliche Fluidöffnungen und einen perforierten oder porösen Dorn, der in das Gehäuse eingeführt und über der Saugstange positioniert werden kann, und

eine Basis,

wobei das Gehäuse eine Ausbuchtung aufweist, die zwischen etwa 30 % und 60 % der Länge des Gehäuses positioniert ist, die die Ausbuchtung bildet.

3. Verwenden einer Abdeckung für eine Zellaussaatkammer bei der Herstellung einer Zellaussaatkammer nach Anspruch 1:

die Abdeckung umfassend eine Öffnung für eine Saugstange, die in das Gehäuse eingeführt werden kann, sowie eine oder mehrere seitliche Fluidöffnungen.

4. Zellaussaatkammer nach Anspruch 1 oder das Verwenden nach Anspruch 2 oder 3, wobei der Dorn ein poröser Dorn ist und eine darin eingesetzte Saugstange aufweist; und/oder wobei die Zellaussaatkammer ferner ein Transplantat oder Gerüst über dem Dorn der Aussaatkammer umfasst.

5. Zellaussaatkammer nach Anspruch 1 oder 4 oder das Verwenden nach einem der Ansprüche 2 bis 4, wobei die Zellaussaatkammer eine seitliche Entlüftungsöffnung aufweist und/oder ein Gerüst zwischen dem Dorn und dem Gehäuse umfasst.

6. Verfahren zum Besiedeln eines Transplantats oder Gerüsts mit Zellen, das Verfahren umfassend:

a) Verbinden der Aussaatkammer nach einem der Ansprüche 1, 4 oder 5 mit einem geschlossenen Einweg-Aussaatsystem, umfassend einen Behälter mit einem Fluid, das Blut oder angereicherte Zellen enthält,
b) Einsetzen eines Transplantats oder Gerüsts über den Dorn der Aussaatkammer,
c) Befüllen der Aussaatkammer mit dem Fluid,
d) Anlegen von Unterdruck an einen unteren Dorn-Auslass und Entleeren der Kammer bis etwa zur Hälfte,
e) Umdrehen der Kammer und
f) Anlegen von Unterdruck und Entleeren der Kammer durch eine obere Auslassöffnung.

7. Verfahren nach Anspruch 6, wobei:

(a) der Unterdruck durch eine Spritze, eine Pumpe oder eine Vakuumquelle erzeugt wird;
(b) das Transplantat oder Gerüst das polymere Transplantat oder Gerüst nach Anspruch 4 oder 5 ist;
(c) das Fluid ein Volumen zwischen etwa 10 ml und 200 ml aufweist;
(d) das Fluid zwischen etwa $10^5$ weißen Blutkörperchen (WBC)/ml und $10^8$ WBC/ml, vorzugsweise zwischen etwa $10^6$ und $10^8$ WBC/ml, noch bevorzugter zwischen etwa $10^6$ und $10^7$ WBC/ml, aufweist;
(e) das Transplantat oder das Gerüst eine Länge zwischen 10 cm und 15 cm aufweist;
(f) die Aussaat innerhalb eines Zeitraums zwischen etwa 1 Minute und 15 Minuten, vorzugsweise zwischen etwa 1 Minute und 10 Minuten, am besten zwischen etwa 1 Minute und 7 Minuten abgeschlossen ist;
(g) das Transplantat oder das Gerüst mit Zellen in einer im Wesentlichen ähnlichen Zelldichte entlang der Länge des Gerüsts ausgesät wird; und/oder
(h) die Zelldichte entlang der Länge des Gerüsts zwischen etwa 0,1 x $10^3$ Zellen/mm$^2$ und $10^5$ Zellen/mm$^2$, einschließlich, vorzugsweise zwischen etwa 0,1 x $10^3$ Zellen/mm$^2$ und $10^4$ Zellen/mm$^2$, einschließlich, entlang der Länge des Gerüsts liegt, am bevorzugtesten zwischen 1 x $10^3$ Zellen/mm$^2$ und $10^4$ Zellen/mm$^2$, einschließlich, entlang der Länge des Gerüsts.

**Revendications**

1. Chambre d'ensemencement cellulaire destinée à être utilisée dans un système fermé jetable d'ensemencement cellulaire, la chambre d'ensemencement cellulaire comprenant :

un boîtier présentant une largeur variable et une longueur,
un capot comprenant une ouverture destinée à l'insertion d'une tige d'aspiration dans le boîtier, et un ou plusieurs orifices latéraux pour fluide, et pour fixer un mandrin perforé ou poreux apte à être disposé autour de la tige d'aspiration, et
une base,
dans laquelle le boîtier présente un renflement disposé entre environ 30 % et 60 % de la longueur du boîtier formant le renflement.

2. Utilisation d'un boîtier destiné à une chambre d'ensemencement cellulaire pour la fabrication d'une chambre d'ensemencement cellulaire selon la revendication 1,

le boîtier présentant une largeur et une longueur variables,
une extrémité ouverte pour recevoir un capot comprenant une ouverture pour l'insertion d'une tige d'aspiration dans le boîtier, un ou plusieurs orifices latéraux pour fluide, et un mandrin perforé ou poreux pouvant être inséré dans le boîtier et disposé autour de la tige d'aspiration, et
une base,
dans laquelle le boîtier présente un renflement disposé entre environ 30 % et 60 % de la longueur du boîtier formant le renflement.

3. Utilisation d'un bouchon pour une chambre d'ensemencement cellulaire dans la production d'une chambre d'ensemencement cellulaire selon la revendication 1 :
le capot comprenant une ouverture destinée à l'insertion d'une tige d'aspiration dans le boîtier, et un ou plusieurs orifices latéraux pour fluide.

4. Chambre d'ensemencement cellulaire selon la revendication 1, ou utilisation selon la revendication 2 ou 3, dans laquelle le mandrin est un mandrin poreux et comprend une tige d'aspiration insérée dans celui-ci ; et/ou dans laquelle la chambre d'ensemencement cellulaire comprend en outre un greffon ou une matrice sur le mandrin de la chambre d'ensemencement.

5. Chambre d'ensemencement cellulaire selon la revendication 1 ou 4, ou utilisation selon l'une quelconque des revendications 2 à 4, dans laquelle la chambre d'ensemencement cellulaire présente un orifice de mise à l'air latéral, et/ou comprend une matrice entre le mandrin et le boîtier.

6. Procédé d'ensemencement d'un greffon ou d'une matrice avec des cellules, le procédé comprenant :

a) le raccordement de la chambre d'ensemencement selon l'une quelconque des revendications 1, 4 ou 5 à un système fermé jetable d'ensemencement comprenant un récipient contenant un fluide comprenant du sang ou des cellules enrichies,
b) l'insertion d'un greffon ou d'une matrice sur le mandrin de la chambre d'ensemencement,
c) le remplissage de la chambre d'ensemencement avec le fluide,
d) l'application d'une pression négative à une sortie inférieure du mandrin et la vidange de la chambre jusqu'à environ la moitié de son volume,
e) l'inversion de la chambre, et
f) l'application d'une pression négative et la vidange de la chambre par une sortie supérieure.

7. Procédé selon la revendication 6, dans lequel :

(a) la pression négative est fournie par une seringue, une pompe ou une source de vide ;
(b) le greffon ou la matrice est le greffon ou la matrice polymérique selon la revendication 4 ou 5 ;
(c) le fluide présente un volume compris entre environ 10 ml et 200 ml ;
(d) le fluide comprend entre environ $10^5$ globules blancs (GB)/ml et $10^8$ GB/ml, de préférence entre environ $10^6$ et $10^8$ GB/ml, de manière davantage préférée entre environ $10^6$ et $10^7$ GB/ml ;
(e) le greffon ou la matrice présente une longueur comprise entre 10 cm et 15 cm ;
(f) l'ensemencement est réalisé dans un intervalle de temps compris entre environ 1 min et 15 min, de préférence entre environ 1 min et 10 min, de manière préférée entre toutes entre environ 1 min et 7 min ;
(g) le greffon ou la matrice est ensemencé(e) avec des cellules à une densité cellulaire sensiblement uniforme le long de la longueur de la matrice ; et/ou
(h) la densité cellulaire est comprise entre environ $0,1 \times 10^3$ cellules/mm$^2$ et $10^5$ cellules/mm$^2$, limites comprises, le long de la longueur de la matrice, de préférence entre environ $0,1 \times 10^3$ cellules/mm$^2$ et $10^4$ cellules/mm$^2$, limites comprises, le long de la longueur de la matrice, de manière préférée entre toutes entre $1 \times 10^3$ cellules/mm$^2$ et $10^4$ cellules/mm$^2$, limites comprises, le long de la longueur de la matrice.

FIG. 1A (Prior Art)

FIG. 1B

FIG.1C

FIG. 1D

FIG.1E

FIG. 1F

FIG. 1G

FIGs. 1B-1G ((Prior Art)

230

600

21

600

180

FIG. 1H (Prior Art)

**FIG. 2A**

**FIG. 2B**

FIG. 3A                    FIG. 3B                    FIG. 3C

EP 4 058 074 B1

**FIG. 3D**

**FIG. 3E**

**FIG. 4A**

FIG. 4B

**FIG. 4C**

**FIG. 5**

**FIG. 6**

FIG. 7A          FIG. 7B          FIG. 7C          FIG. 7D

FIG. 7E          FIG. 7F          FIG. 7G          FIG. 7H

FIG. 8A

FIG. 8B

FIG. 9A

FIG. 9B

**FIG. 9C**

**FIG. 9D**

FIG. 9E

FIG. 9F

**FIG. 10A**

**FIG. 10B**

**FIG. 10C**

**FIG. 10D**

**Inflammation-driven neotissue formation**

FIG. 11A

**Mechano-mediated neotissue remodeling**

FIG. 11B

FIG. 11C

```
┌─────────────────────────────────┐
│  Assessed for eligibility (n=6)  │
└─────────────────────────────────┘
                  │
        ┌─────────────────────┐
        │  Randomized (n=0)   │
        └─────────────────────┘
                  │
            ( Allocation )
                  │
                  ▼
┌─────────────────────────────────────────────────┐
│ Allocated to intervention (n=4)                  │
│  - Received allocated intervention (n=4)         │
│  - Did not receive allocated intervention (n=0)  │
└─────────────────────────────────────────────────┘
                  │
            ( Follow Up )
                  │
                  ▼
┌─────────────────────────────────────────────────┐
│ Lost to follow-up (n=0)                          │
│ Discontinued intervention (n=0)                  │
└─────────────────────────────────────────────────┘
                  │
             ( Analysis )
                  │
                  ▼
┌─────────────────────────────────────────────────┐
│ Analysed (n=4)                                   │
│  - Excluded from analysis (n=0)                  │
└─────────────────────────────────────────────────┘
```

**FIG. 12**

FIG. 13A

## Survival Proportions

**FIG. 13B**

## ☞ ■ Narrowest point by Angiography

**FIG. 14A**

## Narrowest point by IVUS

FIG. 14B

## Narrowest Diameter

6 Week

FIG. 14C

## Max. Pressure Gradient

6 Week

FIG. 14D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 9090863 B
- US 20180353649 A
- US 9090863 B2

### Non-patent literature cited in the description

- **SHIN'OKA et al.** *N. Engl. J. Med.*, 2001, vol. 344 (7), 532-533
- **HIBINO et al.** *J Thorac Cardiovasc Surg.*, vol. 139 (2), 431-436
- **2010** ; **ROH JD et al.** *Biomaterials*, 2008, vol. 29 (10), 1454-1463
- **HIBINO et al.** *FASEB J.*, 2011, vol. 25 (8), 2731-2739
- **HIBINO et al.** *FASEB J.*, 2011, vol. 25 (12), 4253-4263
- **KUROBE et al.** *Tissue Eng Part C Methods*, 2015, vol. 21 (1), 88-93
- **SHIN'OKA et al.** *J Thorac Cardiovasc Surg.*, 2005, vol. 129 (6), 1330-1338
- **HIBINO et al.** *J Thorac Cardiovasc Surg.*, 2010, vol. 139 (2), 431-436
- **FERNANDEZ et al.** *Current opinion in chemical engineering*, 2014, vol. 3, 83-90
- **MCALLISTER et al.** *Lancet*, 2009, vol. 373 (9673), 1440-1446
- **WYSTRYCHOWSKI et al.** *J Vasc Surg*, 2014, vol. 60 (5), 1353-1357
- **PATTERSON et al.** *Regenerative Medicine*, 2012, vol. 7 (3), 409-419
- *Tissue Engineering Part C: Methods.*, 2014, 88-93
- **MATSUMURA et al.** *Biomaterials*, 2003, vol. 24, 2303-8
- **LEE et al.** *J Vis Exp.*, 2014

- **UDELSMAN et al.** *Tissue Eng Part C Methods.*, 2011, vol. 17 (7), 731-736
- **ARRAS et al.** *J Clin Invest*, 1998, vol. 101 (1), 40-50
- **HIBINO et al.** *FASEB J.*, 2011, vol. 25 (12), 4253-63
- **WYNN**. *Nat Rev Immunol*, 2004, vol. 4 (8), 583-94
- **GEISSMANN et al.** *Science*, 2010, vol. 327, 656-661
- **GORDON** ; **TAYLOR**. *Nat. Rev. Immunol.*, 2005, vol. 5, 953-964
- **MANTOVANI et al.** *Trends Immunol.*, 2002, vol. 23, 549-555
- **WYNN** ; **BARRON**. *Semin Liver Dis.*, 2010, vol. 30 (3), 245-257
- **ZIMMERMANN et al.** *PLOS One*, 2010, vol. 5 (6), e11049
- **CHANDRASEKAR et al.** *J Am College Cardiology,*, 2000, vol. 35 (3), 555-562
- **TROJANOWSKA**. *Rheumatology*, 2008, vol. 47, v2-v4
- **FINGERLE et al.** *Proc Natl Acad Sci.*, 1989, vol. 86, 8412
- **CLOWES et al.** *Circ. Res.*, 1985, vol. 56, 139-145
- the Care and Use of Laboratory Animals. ublic Health Service, National Institutes of Health, 2011